# EUROPEAN PATENT APPLICATION

(11) **EP 4 005 577 A1**
(43) Date of publication of application: **01.06.2022**
(21) Application number: 20210058.2
(22) Date of filing: 26.11.2020
(51) Int. Cl.: A61K 35/28, A61K 35/30, A61K 35/32, A61K 35/34, A61K 38/36, A61K 47/34, A61K 47/36, A61K 47/42, A61K 47/69, A61P 29/00, A61P 35/00

(54) **CELLULAR AND/OR EXTRACELLULAR EXTRACTS FOR PREVENTING AND/OR TREATING CANCER AND/OR INFLAMMATION**

(71) Applicant: Novadip Biosciences, 1435 Mont-Saint-Guibert (BE)
(72) Inventor: DUFRANE, Denis, 1380 Lasne (BE)
(74) Representative: Willnegger, Eva

(57) **Abstract**

The present invention relates to a pharmaceutical composition for use in the prevention and/or the treatment of cancer and/or inflammation, comprising (i) a cellular and/or extracellular extract(s) obtained from a scaffold-free 3-dimensional culture of mature cells and a particulate material, wherein the mature cells secreted an extracellular matrix, and wherein the mature cells and the particulate material were embedded in the extracellular matrix; and (ii) a pharmaceutically acceptable excipient.

## Description

### FIELD OF INVENTION

The present invention relates to the field of prevention and/or treatment of cancer. More particularly, the invention relates to the therapeutic use of a pharmaceutical composition comprising a cellular and/or extracellular extract(s) obtained from mature cells, *i.e.* differentiated cells, cultured in a 3-dimensional environment in the presence of a particulate material.

### BACKGROUND OF INVENTION

According to the World Health Organization (WHO), cancer is the second leading cause of death worldwide, accounting for an estimated 9.6 million deaths (one in six deaths) for the sole year 2018. Breast, colorectal, lung, cervical and thyroid cancer are the most common types of cancer among women, whereas lung, prostate, colorectal, stomach and liver cancer are the most frequent cancers among men.

As for prevention, it is estimated that 30% to 50% of deaths associated with cancer could be prevented by modifying or avoiding key risk factors, by early detecting cancer and by early managing patients who develop cancer. For example, limiting or avoiding alcohol and tobacco use, maintaining a healthy weight, in particular through healthy diet and regular exercise, getting proper vaccination against hepatitis B and human papillomavirus (HPV), reducing exposure to ultraviolet radiation and ionizing radiation, managing urban air pollution, among other prevention strategies, may likely limit the occurrence of cancer.

Along with surgery, chemotherapy and radiotherapy strategies have been developed and constitute altogether the current tools to cope with cancer. However, these latter strategies often result in the occurrence of toxic effects for the patient, since the drugs and the rays are not solely destroying cancer tumors but also contribute to the destruction of healthy cells, tissues and organs.

In the last years, expectations were increasingly high regarding to personalized medicine, which aims to propose individualized treatment once the patient's specific biomarkers are identified. However, this strategy is very recent, cost-effective, and the scientific community has not yet been provided with the hindsight needed to assess its effectiveness.

Therefore, there is a need to provide the state of the art with innovative alternative strategies to prevent and manage cancer.

There is also a need to provide the state of the art with less aggressive treatment, *i.e.,* provide treatments that are less toxic and are to be tolerated by the patient's body.

There is further a need to provide the state of the art with active ingredients that originate from natural and/or physiological processes.

### SUMMARY

A first aspect of the invention relates to a pharmaceutical composition for use in the prevention and/or the treatment of cancer and/or inflammation, comprising:
- (i) a cellular and/or extracellular extract(s) obtained from a scaffold-free 3-dimensional culture of mature cells and a particulate material, wherein the mature cells secreted an extracellular matrix, and wherein the mature cells and the particulate material were embedded in the extracellular matrix; and
- (ii) a pharmaceutically acceptable excipient.

In some embodiments, the extracellular extract comprises a matrisomal fraction and/or an exosomal fraction. In certain embodiments, the cellular and/or extracellular extract(s) comprise(s) one or more miRNA(s). In some embodiments, the cells are selected from the group comprising or consisting of primary cells, stem cells, genetically modified cells, and a combination thereof. In certain embodiments, the stem cells are mesenchymal stem cells, preferably adipose tissue-derived stem cells. In some embodiments, the mature cells are selected from the group comprising or consisting of osteoblasts, osteocytes, chondroblasts, chondrocytes, keratinocytes, myofibroblasts, epithelial cells, endothelial cells, adipocytes, neural cells, and precursors thereof. In certain embodiments, the cells secreted OPG.

In some embodiments, the particulate material is selected from the group comprising or consisting of:
- an organic material, including demineralized bone matrix (DBM), gelatin, agar/agarose, alginates chitosan, chondroitin sulfate, collagen, elastin or elastin-like peptides (ELP), fibrinogen, fibrin, fibronectin, proteoglycans, heparan sulfate proteoglycans, hyaluronic acid, polysaccharides, laminins and cellulose derivatives;
- a ceramic material, including particles of calcium phosphate (CaP), calcium carbonate (CaCO₃), calcium sulfate (CaSO₄), or calcium hydroxide (Ca(OH)₂), or combinations thereof;
- a polymer, including polyanhydrides, polylactic acid (PLA), poly(lactic-co-glycolic acid) (PLGA), polyethylene oxide/ polyethylene glycol (PEO/PEG), poly(vinyl alcohol) (PVA), fumarate-based polymers such as, for example poly(propylene fumarate) (PPF) or poly(propylene fumarate-co-ethylene glycol) (P(PF-co-EG)), oligo(poly(ethylene glycol) fumarate) (OPF), poly (n-isopropylacrylamide) (PNIPPAAm), poly(aldehyde guluronate) (PAG), poly(n-vinyl pyrrolidone) (PNVP), or combinations thereof;
- a gel, including a self-assembling oligopeptide gel, a microgel, a nanogel, a particulate gel, a hydrogel, a thixotropic gel, a xerogel, a responsive gel, or combinations thereof;
- a creamer;
and any combination thereof.

In certain embodiments, said particulate material is gelatin, ceramic material, or a demineralized bone matrix (DBM). In some embodiments, the cellular and/or extracellular extract is/are dehydrated and/or sterilized.

In certain embodiments, the cells are autologous, allogeneic, or xenogeneic. In some embodiments, the cancer is a solid cancer. In certain embodiments, the solid cancer is selected from the group comprising, or consisting of, a bone cancer, a brain cancer, a skin cancer, a breast cancer, a cancer of the central nervous system, a cancer of the cervix, a cancer of the upper aero digestive tract, a colorectal cancer, an endometrial cancer, a germ cell cancer, a bladder cancer, a kidney cancer, a laryngeal cancer, a liver cancer, a lung cancer, a neuroblastoma, an esophageal cancer, an ovarian cancer, a pancreatic cancer, a pleural cancer, a prostate cancer, a retinoblastoma, a small intestine cancer, a soft tissue sarcoma, a stomach cancer, a testicular cancer and a thyroid cancer. In some embodiments, the solid cancer is selected from the group comprising, or consisting of, bone cancer, including an osteosarcoma; a brain cancer, including a glioblastoma; and a skin cancer, including a melanoma. In certain embodiments, inflammation is associated with at least one symptom selected in the group consisting of pain, swelling, redness, edema, aching, tenderness, soreness, or any combination thereof; and/or wherein the inflammation is caused by, results in, or contributes to, injury, strain, infection, sprain, trauma, soreness, ache, fatigue, cancer, generalized joint pain, arthritis, osteoarthritis, rheumatoid arthritis, or any combination thereof. In some embodiments, the cancer or inflammation is associated with RANKL/RANK system activation. In certain embodiments, it is combined before use with any one of an isotonic aqueous solution; a scaffold material; another pharmaceutical composition; medical device; a material of biological origin; and any combination thereof. In some embodiments, the said composition is to be formulated as a putty, an emollient, a cream, an ointment, a lotion, a gel, a salve, a controlled-release matrix, a liposomal or a lipid particle preparation, a microcapsule, or a nanocapsule, a suppository, a transdermal delivery system, or any combination thereof.

### DEFINITIONS

Unless otherwise defined, all technical and scientific terms used herein have the same meaning as commonly understood by one of ordinary skill in the art to which this invention pertains. In case of conflict, the present definitions provided by the instant application are to be considered as authentic.

In the present invention, the following terms have the following meanings:
- The term **"about"** preceding a value means plus or less 10% of the value of said value. It is to be understood that the value to which the term "about" refers to is itself also specifically, and preferably, disclosed.
- The term **"comprises"** is intended to mean **"contains", "encompasses"** and **"includes".** In some embodiments, the term **"comprises"** also encompasses the term **"consists of".**
- The term **"cellular extract" is** intended to refer to the content of the cells' material obtained upon disruption of the cell envelope by mechanical, physical and/or chemical force. The cellular extract may or may not comprise all or part of the medium in which the cells were originally cultured and/or maintained. As used herein, a cellular extract may include, but be not limited to, cellular metabolites; cellular nucleic acids, including miRNAs; cellular polypeptides, including transcription factors, growth factors; cellular lipids; and the likes.
- The term **"extracellular extract"** is intended to refer to the content of the cells' material that has been actively and/or passively transported (by, *e.g*., secretion, exocytosis, leakage) out of the cells. As used herein, an extracellular extract may include, but be not limited to, metabolites; nucleic acids, including miRNAs; polypeptides, including transcription factors, growth factors, components of the extracellular matrix; lipids; and the likes.
- The term **"mature cell"** refers to a primary cell, or cell, in particular a stem cell or a genetically modified cell, that has undergone a differentiation process from an undifferentiated state to a differentiated state, including a fully-differentiated state. In some embodiments, **"mature cell"** and **"differentiated cell"** are intended to be equivalent terms.
- The term **"matrisomal fraction"** is intended to refer to a fraction comprising the compounds that compose the extracellular matrix (further referred to as ECM). As used herein, a matrisomal fraction may include, but be not limited to, ECM-polypeptides, ECM-modifying enzymes, ECM-binding growth factors, and other ECM-associated polypeptides.
- The term **"exosomal fraction"** is intended to refer to a fraction comprising extracellular vesicles, *i.e.,* exosomes or exosomes-like vesicles, that are released from cells upon fusion of an intermediate endocytic compartment, the multivesicular body (MVB), with the plasma membrane. In other words, exosomes or exosome-like vesicles correspond to the intraluminal vesicles that are released into the extracellular milieu.
- The term **"miRNA"** or **"miR"** refers to a non-coding RNA of about 18 nucleotides to about 25 nucleotides in length. These miRNAs could originate from multiple origins including: an individual gene encoding a miRNA, from introns of a protein-encoding gene, or from a poly-cistronic transcript that often encodes multiple, closely related miRNAs. In the following disclosure, the standard nomenclature system is applied, in which uncapitalized "mir-X" refers to the pre-miRNA (precursor), and capitalized "miR-X" refers to the mature form. When two mature miRNAs originate from opposite arms of the same pre-miRNA, they are denoted with a "-3p" or a "-5p" suffix. In the following disclosure, unless otherwise specified, the use of the expression miR-X refers to the mature miRNA including both forms -3p and -5p, if any. Within the scope of the invention, the expressions microRNA, miRNA and miR designate the same compound.
- The term **"secretion"** refers to a physiologically active substance transported out of the cell in which it is synthesized. In one embodiment, the physiologically active substance may be any molecule, in particular a protein (such as a growth factor or a transcription factor) or a nucleic acid (such as a miRNA). As used herein, the term **"secretion"** includes both active and passive secretion. In this application, the term **"active secretion"** refers to the secretion of physiologically active substances by living cells, in particular mesenchymal stem cells and preferably adipose tissue-derived stem cells, out of the cells as a response to a stimulus, thereby diffusing into the environment of the cells, such as, *e.g.,* the extracellular matrix. By **"living cells",** it is meant herein cells presenting at least one of the following characteristics: growth and development, reproduction, homeostasis, response to stimuli, consumption, metabolism, excretion. In this application, the term **"passive secretion"** refers to physiologically active substances released by non-living cells or fragments or extracts thereof, out of the cells or fragments or extracts thereof in the absence of a stimulus, thereby diffusing into the environment of the originating cells or fragments or extracts thereof, such as, *e.g.,* the extracellular matrix. By **"non-living cells",** it is meant herein cells presenting none of the following characteristics: growth and development, reproduction, homeostasis, response to stimuli, consumption, metabolism, excretion (non-living cells or fragments or extracts thereof are, for example, dead cells or cellular extracts). Physiologically active substances that are actively or passively secreted may then diffuse into the tissue or organ in which the biomaterial comprising such extracellular matrix is administered. The term **"secretion"** also refers to the level or amount of one or more extracellular extract(s). The amount of extracellular extract secreted by a cell is generally assessed relative to other cells of the population in the frame, or relative to other cells of the plurality. The amount of extracellular extract secreted by a cell can be determined qualitatively or quantitatively, depending on the sensitivity desired for a particular application of the method. An extracellular extract secretion profile can refer to the amount of secretion of one extracellular extract or more than one extracellular extract, such as two or more extracellular extracts, three or more extracellular extracts and four or more extracellular extracts. An exemplary expression of extracellular extract secretion profile for more than one extracellular extract is a ratio representing the amounts of two or more different extracellular extracts. As is described herein below, the amount of an extracellular extract secreted by a cell can include an undetectable level of extracellular extract.
- The term **"treatment", "treating"** or **"alleviation"** refers to therapeutic treatments wherein the object is to prevent or slow down (lessen) cancer or inflammation. Those in need of treatment include those already with the disorder as well as those prone to have the disorder or those in whom cancer or inflammation is to be prevented. A subject is successfully "treated" for cancer or inflammation if, after receiving a therapeutic amount of a pharmaceutical composition according to the present invention, the individual shows observable and/or measurable reduction in, or absence of, one or more of the following: reduction in cancer volume or inflammation zone and/or relief to some extent, one or more of the symptoms associated with cancer or inflammation; reduced morbidity and mortality, and improvement in quality of life issues. The above parameters for assessing successful treatment and improvement in the disorder are readily measurable by routine procedures familiar to a physician.
- The term **"prevention"** refers to preventing or avoiding the occurrence of any symptom of cancer or inflammation. In the present invention, the term "prevention" may refer to a secondary prevention, *i.e.,* to the prevention of the re-occurrence of a symptom or a relapse of cancer or inflammation. It may also refer, when the disease is cancer, to the occurrence of metastases after the treatment and/or the removal of a tumor.
- The term **"therapeutically effective amount"** or **"effective amount"** refers to an amount of the active ingredient(s) that is/are sufficient to promote beneficial or desired results including clinical results. An effective amount can be administered in one or more administration(s).
- The term **"pharmaceutically acceptable vehicle"** refers to a vehicle that does not produce any adverse, allergic or other unwanted reactions when administered to an animal individual, preferably a human individual. It includes any and all solvents, dispersion media, coatings, antibacterial and antifungal agents, isotonic and absorption delaying agents and the like. For human administration, preparations should meet sterility, pyrogenicity, general safety, quality and purity standards as required by regulatory Offices, such as, *e.g.,* the Food and Drug Administration (FDA) in the United States or the European Medicines Agency (EMA) in the European Union.
- The term **"individual"** refers to a vertebrate animal, preferably a mammal, more preferably a human. Examples of individuals include humans, non-human primates, dogs, cats, mice, rats, horses, cows, sheep and transgenic species thereof. In one embodiment, an individual may be a "patient", *i.e.,* a warm-blooded animal, more preferably a human, who/which is awaiting the receipt of, or is receiving medical care or was/is/will be the object of a medical procedure, or is monitored for the development of a disease, in particular cancer or inflammation. In one embodiment, the individual is an adult (for example a human subject above the age of 18). In another embodiment, the individual is a child (for example a human subject below the age of 18). In one embodiment, the individual is a male. In another embodiment, the individual is a female.

Other definitions may appear in context throughout this disclosure.

### DETAILED DESCRIPTION

The inventors have surprisingly observed that extracellular extracts comprising exosomes obtained from adipose tissue-derived stem cells (ASCs) that have been differentiated in differentiation medium and cultured in the presence of a 3-dimension particulate material, possess antiproliferative properties, since they are capable of significantly decreasing the proliferation of osteosarcoma, melanoma and glioblastoma cell lines.

Without wanting to be bound to a theory, the inventors consider that the cellular and/or extracellular extracts obtained from mature 3D-induced cells comprise biologically active ingredients, or a biologically active ingredients cocktail, that may be beneficial for treating and/or preventing cancer and inflammation. Because these biological active ingredients are naturally produced by mature cells, they are believed to provide the tumor and/or the inflammation site with an environment that promote tissue regeneration, and/or tissue repair and/or tissue healing.

The recitation of an embodiment below includes that embodiment as any single embodiment or in combination with any other embodiments or portions thereof and the recited embodiments are applicable to one or more of the aspects recited below. Other features and advantages of the invention will be apparent from the detailed description and from the claims. Thus, other aspects and embodiments of the invention are described in the following disclosure and are within the ambit of the invention.

A first aspect of the invention relates to a pharmaceutical composition for use in the prevention and/or the treatment of cancer and/or inflammation, comprising:
- (i) a cellular and/or extracellular extract(s) obtained from a scaffold-free 3-dimensional culture of mature cells and a particulate material, wherein the mature cells secreted an extracellular matrix, and wherein the mature cells and the particulate material were embedded in the extracellular matrix; and
- (ii) a pharmaceutically acceptable excipient.

In some embodiments, the cellular and/or the extracellular extract(s) is/are biologically active. As used herein, the term "biologically active" is intended to mean that the extracts are capable of eliciting biological responses, such as, *e.g.,* the activation or the inhibition of metabolic and/or regulatory pathways associated with cancer and/or inflammation; the reduction of the proliferation of tumoral and/or inflammatory cells; the healing of cancerous and/or inflamed tissues and/or organs.

As used herein, "scaffold-free culture" is intended to refer to a culture that has not a predefined shape.

As understood herein, a cellular fraction comprises any cellular components, including cellular nucleic acids, cellular metabolites, cellular polypeptides, cellular lipids, and the like.

The cellular extract may be obtained upon separation of the cells from the culture medium and further processing by cell fractionation according to any well-known method from the state of the art, or a method adapted therefrom. Cell fractionation protocols may be found, *e.g.,* in Harris et al. (Cell Biology Protocols; Wiley, 2006); Walker (The Protein Protocols Handbook. Third Edition. 2009; New York (NY): Springer-Verlag New York, LLC); Baghirova et al. (Sequential fractionation and isolation of subcellular proteins from tissue or cultured cells. MethodsX; 2009; Vol. 2, p440-5).

In some embodiments, a cellular extract comprises, but is not limited to, a nuclear fraction, a cytosolic fraction, a membrane fraction, the like, and any mixture thereof.

In some embodiments, the extracellular extract comprises a matrisomal fraction and/or an exosomal fraction.

As used herein, the term "matrisomal fraction" encompasses a fraction that comprises ECM-polypeptides, ECM-modifying enzymes, ECM-binding growth factors, and other ECM-associated polypeptides. In some embodiments, the ECM-polypeptides, ECM-modifying enzymes, ECM-binding growth factors, and other ECM-associated polypeptides are secreted by the cells, in particular the mature cells, when cultured in a proliferation medium or a differentiation medium.

In practice, the matrisomal fraction may be isolated by sequential extractions of fresh or frozen samples of scaffold-free 3-dimensional culture of mature cells and a particulate material accordingly to the method from the state of the art, or a method adapted therefrom, the overall strategy being to sequentially remove (1) cytosolic proteins (2), nuclear proteins (3), membrane proteins (4), and cytoskeletal proteins in order to obtain an insoluble fraction enriched for ECM proteins. Illustratively, one may use the CNMCS (Cytosol/Nucleus/Membrane/Cytoskeleton) Compartmental Protein Extraction kit (Cytomol^{®}, Union City, CA) according to manufacturer's instructions.

As used herein, the term "exosomal fraction" encompasses a fraction that comprises exosomes or exosome-like vesicles.

In practice, the term "exosome" refers to endocytic-derived nanovesicles that are secreted by nearly all cell types in the body. The exosomes or exosome-like vesicles comprise proteins, nucleic acids, in particular miRNAs, and lipids. In practice, the exosomes or exosome-like vesicles may be isolated and/or purified according to any suitable method known in the state of the art, or a method adapted therefrom. Illustratively, the exosome fraction may be isolated by differential centrifugation from culture medium; by polymer precipitation; by high-performance liquid chromatography (HPLC). Non-limitative example of differential centrifugation method from culture medium may include the following steps:
- Centrifugation for 10-20 min at a speed of about 300×g to about 500×g, so as to remove cells;
- Centrifugation for 10-20 min at a speed of about 1,500×g to about 3,000×g, so as to remove dead cells;
- Centrifugation for 20-45 min at a speed of about 7,500×g to about 15,000×g, so as to remove cell debris;
- One or more ultracentrifugation for 30-120 min at a speed of about 100,000×g to about 200,000×g, so as to pellet the exosomes.

Alternative methods to isolate exosomes may take advantage of commercial kits, such as, *e.g.,* the exoEasy Maxi Kit (Qiagen^{®}) or the Total Exosome Isolation Kit (ThermoFisher Scientific^{®}).

In some embodiments, the exosomes or the exosome-like vesicles have an average diameter ranging from about 25 nm to about 150 nm, preferably from about 30 nm to 120 nm. Within the scope of the instant invention, the expression "from about 25 nm to about 150 nm" includes 25, 30, 35, 40, 45, 50, 55, 60, 65, 70, 75, 80, 85, 90, 95, 100, 105, 110, 115, 120, 125, 130, 135, 140, 145 and 150 nm.

In practice, the measure of the mean sizes and diameters of particles may be performed by any suitable methods known in the state of the art, or a method adapted therefrom. Non-limiting examples of such methods include atomic force microscopy (AFM), transmission electron microscopy (TEM), scanning electron microscopy (SEM), dynamic light scattering (DLS).

In certain embodiments, the cellular and/or an extracellular extract(s) comprise(s) one or more miRNA(s).

As used herein, the term "one or more" encompasses 1, 2, 3, 4, 5, 6, 7, 8, 9, 10, 11, 12, 13, 14, 15, 16, 17, 18, 19, 20, 22, 24, 26, 28, 30, 32, 34, 36, 38, 40, 50, or more. In some embodiments, "one or more" also encompasses at least 1, 2, 3, 4, 5, 6, 7, 8, 9, 10, 11, 12, 13, 14, 15, 16, 17, 18, 19, 20, 22, 24, 26, 28, 30, 32, 34, 36, 38, 40, 50.

Criteria and conventions for miRNA identification and nomenclature have been described in Ambros et al. (A uniform system for microRNA annotation. RNA 2003 9(3):277-279). The miRNAs sequences may be easily retrieved from the miRbase database (http://www.mirbase.org/) or the miRDB database (http://www.mirdb.org/).

In practice, the RNAs content of the cellular and/or an extracellular extract(s) according to the instant invention may be assessed by any suitable method known in the art, or any method adapted therefrom. Illustratively, RNA may be extracted, e.g. by the mean of commercial kit (such as miRNeasy kit from Qiagen^{®}); and further sequenced, e.g. by the mean of a high-throughput sequencing system (such as NextSeq 500 system from Illumina^{®}). Illustratively, one may use the Qiazol lysis reagent (Qiagen^{®}, Hilden, Germany) and a Precellys homogenizer (Bertin^{®} instruments, Montigny-le-Bretonneux, France). RNAs may be purified using Rneasy mini kit (Qiagen^{®}, Hilden, Germany) with an additional on column DNase digestion according to the manufacturer's instruction.

Quality and quantity of RNA may be determined using a spectrophotometer (Spectramax^{®} 190, Molecular Devices^{®}, California, USA). cDNA may be synthesized from 0.5/µg of total RNA using RT² RNA first strand kit (Qiagen^{®}, Hilden, Germany) for genes expression profiles though customized PCR arrays (Customized Human Osteogenic and angiogenic RT² Profiler Assay - Qiagen^{®}, Hilden, Germany). The ABI Quantstudio 5 system (Applied Biosystems^{®}) and SYBR Green ROX Mastermix (Qiagen^{®}, Hilden, Germany) may be used for detection of the amplification product. Quantification may be obtained according to the ΔΔCT method. The final result of each sample may be normalized to the means of expression level of housekeeping genes (*e.g.* ACTB, B2M and GAPDH).

In practice, cellular miRNAs may be isolated by any suitable method known from the state of the art, or a method adapted therefrom. One may refer, *e.g.,* to Chapter 7: Extraction, Purification, and Analysis of mRNA from Eukaryotic Cells of Molecular Cloning: a laboratory manual (Russell and Sambrook; 2001; Cold Spring Harbor Laboratory). Illustratively, miRNAs may be isolated by a commercial kit, such as, *e.g.,* RNeasy Mini kit (Qiagen^{®}) or MagMax mirVana Total RNA isolation kit (Applied Biosystems^{®}).

In some embodiments, the cellular extract comprises one or more miRNA(s) selected in a group comprising, or consisting of, hsa-miR-210-3p, hsa-miR-409-3p, hsa-let-7a-5p, hsa-miR-29b-3p, hsa-miR-30e-3p, hsa-let-7b-5p, hsa-miR-3184-3p, hsa-miR-92a-3p, hsa-miR-320a, hsa-miR-24-3p, hsa-let-7d-5p, hsa-miR-193b-5p, hsa-miR-361-3p, hsa-miR-199a-5p, hsa-miR-25-3p, hsa-miR-181a-5p, hsa-miR-151a-3p, hsa-miR-214-3p, hsa-miR-193a-5p, hsa-miR-30c-5p, hsa-miR-154-5p, hsa-let-7f-5p, hsa-miR-199a-3p, hsa-miR-664b-3p, hsa-miR-664a-5p, hsa-miR-3607-5p, hsa-miR-29a-3p, hsa-miR-27a-3p, hsa-miR-92b-3p, hsa-miR-199b-3p, hsa-miR-342-3p, hsa-miR-320b, hsa-miR-1291, hsa-let-7e-5p, hsa-miR-130a-3p, hsa-miR-3651, hsa-miR-103b, hsa-miR-1273g-3p, hsa-miR-30a-3p, hsa-miR-664b-5p, hsa-miR-34a-3p, hsa-miR-125a-5p, hsa-miR-145-5p, hsa-miR-664a-3p, hsa-miR-140-5p, hsa-miR-21-5p, hsa-miR-28-3p, hsa-miR-98-5p, hsa-miR-3609, hsa-let-7i-5p, hsa-miR-93-5p, hsa-miR-146b-5p, hsa-miR-374c-3p, hsa-miR-125b-5p, hsa-miR-34a-5p, hsa-miR-337-3p, hsa-miR-10a-5p, hsa-let-7g-5p, hsa-miR-222-3p, hsa-miR-4449, hsa-miR-22-3p, hsa-miR-191-5p, hsa-miR-3074-5p, hsa-miR-6516-3p, hsa-miR-4668-5p, hsa-miR-574-3p, hsa-miR-424-5p, hsa-let-7i-3p, hsa-miR-24-2-5p, hsa-miR-199b-5p, hsa-miR-424-3p, hsa-miR-103a-3p, hsa-miR-29b-1-5p, hsa-miR-423-5p, hsa-miR-328-3p, hsa-miR-324-5p, hsa-miR-335-5p, hsa-miR-574-5p, hsa-miR-17-5p, hsa-miR-660-5p, hsa-miR-425-5p, hsa-miR-23b-3p, hsa-miR-23a-3p, hsa-miR-185-5p, hsa-miR-4461, hsa-miR-196a-5p, hsa-let-7d-3p, hsa-miR-374b-5p, hsa-miR-127-3p, hsa-let-7c-5p, hsa-miR-423-3p, hsa-miR-196b-5p, hsa-miR-221-3p, hsa-miR-382-5p, hsa-miR-619-5p, hsa-miR-3613-5p, hsa-miR-3653-5p, hsa-miR-19b-3p, hsa-miR-99b-5p, hsa-miR-376c-3p, hsa-miR-99b-3p, hsa-miR-663b, hsa-miR-495-3p, hsa-miR-454-3p, and a combination thereof. In practice, said cellular extract may be obtained from a scaffold-free 3-dimensional culture of mature cells, in particular osteogenic differentiated adipose tissue-derived stem cells and gelatin.

In certain embodiments, the one or more miRNA(s) is/are selected in a group comprising hsa-miR-210-3p, hsa-miR-409-3p, hsa-miR-361-3p, hsa-miR-130a-3p, hsa-miR-660-5p, hsa-miR-199b-5p, hsa-miR-3074-5p, hsa-let-7i-5p, hsa-miR-24-3p, hsa-miR-342-3p, hsa-miR-214-3p, hsa-miR-199a-5p, hsa-miR-3607-5p, hsa-miR-221-3p, hsa-miR-4449, hsa-miR-382-5p, hsa-miR-196b-5p, hsa-miR-663a, hsa-miR-4485-3p, hsa-miR-6723-5p and a combination thereof.

In one embodiment, the cellular extract comprises one or more miRNA(s) selected in a group comprising, or consisting of, hsa-miR-210-3p, hsa-miR-409-3p, hsa-let-7a-5p, hsa-let-7b-5p, hsa-miR-24-3p, hsa-let-7f-5p, hsa-miR-199a-5p, hsa-miR-214-3p, hsa-miR-3607-5p, hsa-miR-125a-5p, hsa-miR-199b-3p, hsa-miR-125b-5p, hsa-miR-21-5p, hsa-let-7e-5p, hsa-let-7i-5p, hsa-let-7g-5p, hsa-miR-574-3p, hsa-miR-574-5p, hsa-miR-191-5p, hsa-miR-196a-5p, hsa-miR-221-3p, hsa-miR-25-3p, hsa-miR-423-5p, hsa-miR-1273g-3p, hsa-let-7d-5p, hsa-miR-199b-5p, hsa-miR-199a-3p, hsa-miR-193a-5p, hsa-miR-3184-3p, hsa-miR-3653-5p, hsa-miR-342-3p, hsa-miR-28-3p, hsa-miR-23b-3p, hsa-let-7c-5p, hsa-miR-222-3p, hsa-miR-29a-3p, hsa-miR-92a-3p, hsa-miR-30a-3p, hsa-miR-424-3p, hsa-miR-423-3p, hsa-miR-34a-5p, hsa-miR-424-5p, hsa-miR-145-5p, hsa-miR-328-3p, hsa-miR-3074-5p, hsa-let-7d-3p, hsa-miR-93-5p, hsa-miR-23a-3p, hsa-miR-19b-3p, hsa-miR-146b-5p, hsa-miR-320b, hsa-miR-337-3p, hsa-miR-17-5p, hsa-miR-130a-3p, hsa-miR-193b-5p, hsa-miR-382-5p, hsa-miR-30c-5p, hsa-miR-98-5p, hsa-miR-664a-3p, hsa-miR-92b-3p, hsa-miR-4449, hsa-miR-320a, hsa-miR-181a-5p, hsa-miR-3651, hsa-miR-185-5p, hsa-miR-664b-5p, hsa-miR-196b-5p, hsa-miR-27a-3p, hsa-miR-29b-3p, hsa-miR-664b-3p, hsa-miR-99b-5p, hsa-miR-103a-3p, hsa-miR-6516-3p, hsa-miR-22-3p, hsa-miR-26a-5p, hsa-miR-103b, hsa-miR-1291, hsa-miR-425-5p, hsa-miR-22-5p, hsa-miR-374c-3p, hsa-let-7i-3p, hsa-miR-374b-5p, hsa-miR-455-3p, hsa-miR-532-3p, hsa-miR-619-5p, hsa-miR-28-5p, hsa-miR-10a-5p, hsa-miR-151a-3p, hsa-miR-30e-3p, hsa-miR-324-5p, hsa-miR-495-3p, hsa-miR-576-5p, hsa-miR-625-3p, hsa-miR-671-5p, hsa-miR-1271-5p, hsa-miR-186-5p, hsa-miR-23a-5p, hsa-miR-3613-5p, hsa-miR-376c-3p, hsa-miR-4461, hsa-miR-454-3p, hsa-miR-6724-5p, hsa-let-7b-3p, hsa-miR-190a-5p, hsa-miR-26b-3p, hsa-miR-3609, hsa-miR-411-5p, hsa-miR-425-3p, hsa-miR-4485-3p and a mixture thereof. In practice, said cellular extract may be obtained from a scaffold-free 3-dimensional culture of mature cells, in particular osteogenic differentiated adipose tissue-derived stem cells and HA/β-TCP.

In certain embodiments, the one or more miRNA(s) is/are selected in a group comprising hsa-miR210-3p, hsa-miR-409-3p, hsa-let-7i-5p, hsa-miR-24-3p, hsa-miR-93-5p, hsa-miR-382-5p, hsa-miR-4485-3p, and a combination thereof.

In some embodiments, the extracellular extract comprises one or more miRNA(s) selected in a group comprising, or consisting of, hsa-miR-210-3p, hsa-miR-409-3p, hsa-let-7a-5p, hsa-miR-92a-3p, hsa-miR-92b-3p, hsa-miR-24-2-5p, hsa-let-7b-5p, hsa-miR-125b-5p, hsa-miR-335-5p, hsa-miR-26a-2-3p, hsa-let-7f-5p, hsa-miR-337-3p, hsa-let-7f-1-3p, hsa-miR-301a-3p, hsa-miR-24-3p, hsa-miR-93-5p, hsa-miR-196b-5p, hsa-miR-98-3p, hsa-miR-21-5p, hsa-miR-3613-3p, hsa-miR-1273a, hsa-miR-23b-3p, hsa-miR-199a-3p, hsa-miR-23a-5p, hsa-miR-28-5p, hsa-miR-1273g-3p, hsa-miR-145-5p, hsa-miR-374b-5p, hsa-miR-34a-3p, hsa-miR-574-3p, hsa-miR-30a-3p, hsa-miR-660-5p, hsa-miR-425-3p, hsa-miR-25-3p, hsa-miR-382-5p, hsa-miR-186-5p, hsa-miR-505-3p, hsa-let-7e-5p, hsa-miR-19b-3p, hsa-miR-454-3p, hsa-miR-34b-3p, hsa-miR-214-3p, hsa-miR-10a-5p, hsa-miR-361-3p, hsa-miR-199a-5p, hsa-miR-619-5p, hsa-miR-495-3p, hsa-miR-10b-5p, hsa-miR-196a-5p, hsa-miR-17-5p, hsa-miR-425-5p, hsa-miR-1306-5p, hsa-miR-199b-5p, hsa-miR-193a-5p, hsa-miR-2053, hsa-miR-22-5p, hsa-miR-221-3p, hsa-miR-320b, hsa-miR-5096, hsa-miR-378a-3p, hsa-miR-424-5p, hsa-miR-193b-5p, hsa-miR-494-3p, hsa-miR-411-5p, hsa-miR-23a-3p, hsa-miR-320a, hsa-miR-27a-3p, hsa-miR-505-5p, hsa-let-7c-5p, hsa-miR-151a-3p, hsa-miR-4449, hsa-miR-664a-3p, hsa-miR-199b-3p, hsa-let-7a-3p, hsa-miR-532-3p, hsa-miR-26a-5p, hsa-miR-191-5p, hsa-miR-30e-3p, hsa-miR-532-5p, hsa-miR-377-3p, hsa-miR-574-5p, hsa-miR-22-3p, hsa-miR-126-5p, hsa-miR-485-3p, hsa-miR-424-3p, hsa-miR-99b-5p, hsa-miR-30c-5p, hsa-miR-590-3p, hsa-miR-423-5p, hsa-miR-625-3p, hsa-miR-130b-3p, hsa-miR-99a-3p, hsa-miR-342-3p, hsa-miR-4668-5p, hsa-miR-136-3p, hsa-miR-143-3p, hsa-let-7d-3p, hsa-miR-29b-3p, hsa-miR-15b-3p, hsa-miR-26b-3p, hsa-miR-130a-3p, hsa-miR-423-3p, hsa-miR-29b-1-5p, hsa-miR-3607-5p, hsa-miR-3184-3p, hsa-miR-376c-3p, hsa-miR-99b-3p, hsa-miR-3651, hsa-miR-222-3p, hsa-let-7b-3p, hsa-miR-127-3p, hsa-miR-374a-3p, hsa-let-7g-5p, hsa-miR-3074-5p, hsa-miR-134-5p, hsa-miR-376a-3p, hsa-miR-125a-5p, hsa-miR-98-5p, hsa-miR-324-5p, hsa-miR-485-5p, hsa-let-7d-5p, hsa-miR-185-5p, hsa-miR-3605-3p, hsa-miR-103b, hsa-miR-29a-3p, hsa-miR-19a-3p, hsa-miR-101-3p, hsa-miR-126-3p, hsa-let-7i-5p, hsa-miR-34a-5p, hsa-miR-103a-3p, hsa-miR-149-5p, hsa-miR-146b-5p, hsa-miR-374c-3p, hsa-miR-1246, hsa-miR-193b-3p, hsa-miR-4454, hsa-miR-181a-5p, hsa-miR-138-5p, hsa-miR-223-3p, hsa-miR-28-3p, hsa-miR-328-3p, hsa-miR-190a-5p, hsa-miR-340-3p, hsa-miR-874-3p, hsa-miR-7847-3p, hsa-miR-6724-5p, hsa-miR-369-5p, and a combination thereof. In practice, said extracellular extract may be obtained from a scaffold-free 3-dimensional culture of mature cells, in particular osteogenic differentiated adipose tissue-derived stem cells and gelatin.

In some embodiments, the one or more miRNA(s) is/are selected in a group comprising hsa-miR-210-3p, hsa-miR-409-3p, hsa-let-7i-5p, hsa-miR-3607-5p, hsa-let-7a-3p, hsa-miR-1246, hsa-miR-335-5p, hsa-miR-4454, hsa-miR-181a-5p, hsa-miR-374c-3p, hsa-miR-619-5p, hsa-miR-29b-3p, hsa-let7e-5p, hsa-miR-23b-3p, hsa-miR-4449, hsa-miR-663a, hsa-miR-25-3p, hsa-let-7b-3p, hsa-miR-138-5p, hsa-miR-3613-3p, hsa-miR-6516-3p, hsa-miR-664a-3p, hsa-miR-3648, hsa-miR-3653-5p, hsa-miR-6516-5p, hsa-miR-3651, hsa-miR-3687, hsa-miR-664-5p, hsa-miR-664-3p, and a combination thereof.

In certain embodiments, the extracellular extract comprises one or more miRNA(s) selected in a group comprising, or consisting of, hsa-miR-210-3p, hsa-miR-409-3p, hsa-let-7a-5p, hsa-let-7b-5p, hsa-miR-24-3p, hsa-miR-21-5p, hsa-let-7f-5p, hsa-miR-574-3p, hsa-miR-23b-3p, hsa-miR-1273g-3p, hsa-miR-25-3p, hsa-miR-199a-5p, hsa-miR-196a-5p, hsa-miR-214-3p, hsa-miR-125a-5p, hsa-miR-221-3p, hsa-miR-222-3p, hsa-let-7e-5p, hsa-miR-191-5p, hsa-miR-199b-3p, hsa-miR-342-3p, hsa-miR-23a-3p, hsa-miR-424-3p, hsa-miR-28-3p, hsa-let-7g-5p, hsa-miR-92a-3p, hsa-miR-424-5p, hsa-let-7d-3p, hsa-miR-4454, hsa-miR-146b-5p, hsa-miR-423-5p, hsa-miR-29a-3p, hsa-miR-574-5p, hsa-miR-199b-5p, hsa-miR-125b-5p, hsa-miR-3184-3p, hsa-let-7c-5p, hsa-miR-337-3p, hsa-let-7d-5p, hsa-miR-145-5p, hsa-miR-93-5p, hsa-miR-619-5p, hsa-miR-130a-3p, hsa-let-7i-5p, hsa-miR-199a-3p, hsa-miR-30a-3p, hsa-miR-320b, hsa-miR-193a-5p, hsa-miR-382-5p, hsa-miR-423-3p, hsa-miR-17-5p, hsa-miR-19b-3p, hsa-miR-92b-3p, hsa-miR-320a, hsa-miR-3074-5p, hsa-miR-376c-3p, hsa-let-7b-3p, hsa-miR-625-3p, hsa-miR-99b-5p, hsa-miR-34a-5p, hsa-miR-5096, hsa-miR-30e-3p, hsa-miR-22-3p, hsa-miR-151a-3p, hsa-miR-186-5p, hsa-miR-193b-5p, hsa-miR-328-3p, hsa-miR-4449, hsa-miR-27a-3p, hsa-miR-30c-5p, hsa-miR-494-3p, hsa-miR-98-5p, hsa-miR-10a-5p, hsa-miR-29b-3p, hsa-miR-374b-5p, hsa-miR-335-5p, hsa-miR-374c-3p, hsa-miR-425-5p, hsa-miR-181a-5p, hsa-miR-196b-5p, hsa-let-7f-1-3p, hsa-miR-4668-5p, hsa-miR-660-5p, hsa-miR-664a-3p, hsa-miR-185-5p, hsa-miR-3651, hsa-miR-495-3p, hsa-let-7a-3p, hsa-miR-28-5p, hsa-miR-99b-3p, hsa-miR-103a-3p, hsa-miR-19a-3p, hsa-miR-126-5p, hsa-miR-2053, hsa-miR-29b-1-5p, hsa-miR-3648, hsa-miR-374a-3p, hsa-miR-454-3p, hsa-miR-532-3p, hsa-miR-136-3p, hsa-miR-361-3p, hsa-miR-1246, hsa-miR-130b-3p, hsa-miR-134-5p, hsa-miR-154-5p, hsa-miR-34a-3p, hsa-miR-576-5p, hsa-miR-874-3p, hsa-miR-100-5p, hsa-miR-103b, hsa-miR-1273a, hsa-miR-1306-5p, hsa-miR-138-5p, hsa-miR-15b-3p, hsa-miR-26b-3p, hsa-miR-10b-5p, hsa-miR-22-5p, hsa-miR-3613-3p, hsa-miR-655-3p, hsa-miR-7-1-3p, hsa-miR-23a-5p, hsa-miR-24-2-5p, hsa-miR-3605-3p, hsa-miR-6832-3p, hsa-miR-146a-5p, hsa-miR-16-2-3p, hsa-miR-181b-5p, hsa-miR-26a-2-3p, hsa-miR-376a-3p, hsa-miR-539-5p, hsa-miR-708-5p, hsa-miR-98-3p, hsa-miR-1237-5p, hsa-miR-223-3p, hsa-miR-532-5p, hsa-miR-542-3p, hsa-miR-663a, hsa-miR-101-3p, hsa-miR-143-3p, hsa-miR-21-3p, hsa-miR-224-5p, hsa-miR-26a-5p, hsa-miR-27a-5p, hsa-miR-324-5p, hsa-miR-340-3p, hsa-miR-379-5p, hsa-miR-409-5p, hsa-miR-543, hsa-miR-5787, hsa-miR-6089, hsa-miR-127-3p, hsa-miR-149-5p, hsa-miR-181c-5p, hsa-miR-193b-3p, hsa-miR-222-5p, hsa-miR-3613-5p, hsa-miR-365b-3p, hsa-miR-3960, hsa-miR-485-3p, hsa-miR-6087, hsa-miR-92a-1-5p and a mixture thereof. In practice, said extracellular extract may be obtained from a scaffold-free 3-dimensional culture of mature cells, in particular osteogenic differentiated adipose tissue-derived stem cells and HA/β-TCP.

In some embodiments, the one or more miRNAs is/are selected in a group comprising hsa-miR210-3p, hsa-miR-409-3p, hsa-miR-4454, hsa-miR-619-5p, hsa-miR-3607-5p, hsa-miR-3613-3p, hsa-miR-664b-5p, hsa-miR-3687, hsa-miR-3653-5p, hsa-miR-664b-3p, and a combination thereof.

In practice, examples of scaffold-free 3-dimensional cultures of mature cells, *i.e.,* differentiated cells, in the presence of a particulate material, have been disclosed and characterized in the publications WO2019/057862 and WO2020/058511, which are incorporated herein by reference.

In some embodiments, scaffold-free 3-dimensional cultures of mature cells may be obtained by:
- (1) contacting viable cells with a particulate material, so as to obtain a first combination;
- (2) culturing the first combination obtained in step (1) in a culture medium such that the cells secrete an extracellular matrix, and wherein the cells and the particulate material are embedded in the extracellular matrix, so as to form a 3-dimensional structure.

As used herein, the expression "viable cells" refers to a population of primary cells or differentiated cells, including differentiated stem cells or differentiated genetically modified cells, that possesses proliferative properties and/or active metabolic properties.

As used herein, the term "embedded in" is intended to mean "enclosed closely in" or "being an integral part of'. In other words, by "cells and the particulate material are embedded in the extracellular matrix", one may understand that the cells, the particulate material and the extracellular matrix are intimately linked one to another and that the three ingredients make one unique structure (or network).

In certain embodiments, the cells are selected from the group comprising or consisting of primary cells, stem cells, genetically modified cells, and a combination thereof.

In practice, the cells according to the instant invention may be animal cells, preferably mammal cells, more preferably human cells.

The cells are autologous, allogeneic, or xenogeneic.

In some embodiments, primary cells may be selected in a group comprising or consisting of osteocytes, osteoblasts, osteoclasts, chondroblasts, chondrocytes, keratinocytes, dermal fibroblasts, fibroblasts, epithelial cells, hematopoietic cells, hepatic cells, neuronal cells, myofibroblasts, endothelial cells, adipocytes, and a combination thereof.

In certain embodiments, primary cells may be selected in a group comprising osteocytes, osteoblasts, osteoclasts, chondroblasts, chondrocytes and a mixture combination thereof. Because primary cells are differentiated cells, they can be cultured in any suitable culture medium for maintenance or proliferation purposes. In some embodiments, the primary cells may be cultured in a culture medium suitable for allowing proliferation or maintenance of the cells.

In certain embodiments, stem cells may be selected in a group comprising osteoprogenitors, embryonic stem cells (ESCs), mesenchymal stem cells (MSCs), pluripotent stem cells (pSCs) and induced pluripotent stem cells (ipSCs).

As used herein, "embryonic stem cells" (ESCs) generally refer to embryonic cells, which are capable of differentiating into cells of any one of the three embryonic germ layers, namely endoderm, ectoderm or mesoderm, or capable of being maintained in an undifferentiated state. Such cells may comprise cells which are obtained from the embryonic tissue formed after gestation (*e.g*., blastocyst) before implantation of the embryo (*i.e.,* a pre-implantation blastocyst), extended blastocyst cells (EBCs) which are obtained from a post-implantation/pre-gastrulation stage blastocyst (see, *e.g.,* WO2006/040763), embryonic germ (EG) cells which are obtained from the genital tissue of a fetus any time during gestation, preferably before 10 weeks of gestation and other methods with non-fertilized eggs, such as parthenogenesis method or nuclear transfer.

In certain embodiments, the ESCs according to the invention are animal ESCs, preferably mammal ESCs, more preferably human ESCs (hESCs).

In practice, suitable EScs may be obtained using well-known cell-culture methods. For example, ESCs can be isolated from blastocysts. Blastocysts are typically obtained from *in vivo* preimplantation embryos or from *in vitro* fertilized (IVF) embryos. Alternatively, a single cell embryo can be expanded to the blastocyst stage. Further details on methods of preparation ESCs may be found in U.S. Pat. No. 5,843,780.

In some embodiments, hESCs may advantageously be obtained without embryo destruction, as described by Chung *et al.* (2008)). In some embodiments, hESCs may be advantageously obtained from embryo collected or isolated less than 14 days upon fertilization. In some embodiments, the ESCs are not human ESCs.

As used herein, "mesenchymal stem cells" (MSCs) generally refer to stromal cells from a specialized tissue (also named differentiated tissue) and capable of self-renewal (*i.e.,* making identical copies of themselves) for the lifetime of the organism and have multipotent differentiation potential.

In some embodiments, the MSCs according to the invention are animal MSCs, preferably mammal MSCs, more preferably human MSCs (hMSCs). In practice, hMSCs suitable for implementing the instant invention thus encompass any suitable human multipotent stem cells derived from any suitable tissue, using any appropriate isolation method. Illustratively, hMSCs encompass, but are not limited to, adult multilineage inducible (MIAMI) cells (D'Ippolito *et al.;* 2004), cord blood derived stem cells (Kogler *et al.;* 2004), mesoangioblasts (Sampaolesi *et al.;* 2006; Dellavalle *et al.;* 2007), and amniotic stem cells (De Coppi *et al.;* 2007). Furthermore, umbilical cord blood banks (*e.g.,* Etablissement Français du Sang, France) provide secure and easily available sources of such cells for transplantation.

In some embodiments, the MSCs are pre-osteoblasts, pre-chondroblasts, pre-keratinocytes, pre-fibroblasts. In some embodiments, the MSCs according to the invention are pre-osteoblasts or pre-chondroblasts.

In some embodiments, the stem cells are mesenchymal stem cells, preferably adipose tissue-derived stem cells (ASCs).

As used herein, the following terms are considered to refer to ASCs: Adipose-derived Stem/Stromal Cells (ASCs); Adipose Derived Adult Stem (ADAS) Cells, Adipose Derived Adult Stromal Cells, Adipose Derived Stromal Cells (ADSC), Adipose Stromal Cells (ASC), Adipose Mesenchymal Stem Cells (AdMSC), Lipoblasts, Pericytes, Pre-Adipocytes, Processed Lipoaspirate (PLA) Cells.

In one embodiment, ASCs are of animal origin, preferably of mammal origin, more preferably of human origin. Accordingly, in one embodiment, ASCs are animal ASCs, preferably mammal ASCs, more preferably human ASCs. In a preferred embodiment, ASCs are human ASCs.

Methods of isolating stem cells from adipose tissue are known in the art and are disclosed for example in Zuk et al. (Tissue Engineering. 2001, 7:211-228). In one embodiment, ASCs are isolated from adipose tissue by liposuction.

As an illustration, adipose tissue may be collected by needle biopsy or liposuction aspiration. ASCs may be isolated from adipose tissue by first washing the tissue sample extensively with phosphate-buffered saline (PBS), optionally containing antibiotics, for example 1% Penicillin/Streptomycin (P/S). Then the sample may be placed in a sterile tissue culture plate, or a sterile tube, with collagenase for tissue digestion (for example, Collagenase Type I prepared in PBS containing 2% P/S), and incubated for 60 min at 37°C, 5% CO₂, in a water bath, with manual shaking every 20 min. The collagenase activity may be neutralized by adding culture medium (for example DMEM containing 10% human platelet lysate (hPL)). Upon disintegration, the sample may be transferred to a tube. The stromal vascular fraction (SVF), containing the ASCs, is obtained by centrifuging the sample (for example at 2,000 rpm for 5 min). To complete the separation of the stromal cells from the primary adipocytes, the sample may be shaken vigorously to thoroughly disrupt the pellet and to mix the cells. The centrifugation step may be repeated. After spinning and the collagenase solution aspirate, the pellet may be resuspended in lysis buffer, incubated on ice (for example for 10 min), washed (for example with PBS/2% P/S) and centrifuged (for example at 2,000 rpm for 5 min). The supernatant may be then aspirated, the cell pellet resuspended in medium (for example, stromal medium, i.e. α-MEM, supplemented with 20% FBS, 1% L-glutamine, and 1% P/S), and the cell suspension filtered (for example, through 70 µm cell strainer). The sample containing the cells may be finally plated in culture plates and incubated at 37°C, 5% CO₂.

In some embodiments, ASCs of the invention are isolated from the stromal vascular fraction of adipose tissue. In some embodiments, the lipoaspirate may be kept several hours at room temperature, or at +4°C for 24-72 hours prior to use, or below 0°C, for example -18°C or -80°C, for long-term conservation.

In certain embodiments, ASCs may be fresh ASCs or refrigerated ASCs. Fresh ASCs are isolated ASCs which have not undergone a refrigerating treatment. Refrigerated ASCs are isolated ASCs which have undergone a refrigerating treatment. In some embodiments, a refrigerating treatment means any treatment below 0°C. In certain embodiments, the refrigerating treatment may be performed at about -18°C, at -80°C or at -180°C. In one specific embodiment, the refrigerating treatment may be cryopreservation.

As used herein, the term "pluripotent" refers to cells having the capacity to generate a cellular progeny that can undergo differentiation, under appropriate conditions, into cell types that collectively exhibit characteristics associated with cell lineages from the three germ layers (endoderm, mesoderm, and ectoderm). Pluripotent stem cells can contribute to tissues of a prenatal, postnatal or adult organism. A standard art-accepted test, such as the ability to form a teratoma in 8 to 12 weeks-old SCID mice, can be used to establish the pluripotency of a cell population. However, identification of various pluripotent stem cell characteristics can also be used to identify pluripotent cells. In some embodiments of the invention, the pluripotent stem cells are animal pluripotent stem cells, preferably mammal pluripotent stem cells, more preferably human pluripotent stem cells.

As used herein, an "induced pluripotent stem cell" (iPSC) refers to a pluripotent stem cell artificially derived from a non-pluripotent cell. A non-pluripotent cell may be a cell of lesser ability (or potency) to self-renew and to differentiate as compared to a pluripotent stem cell. Cells of lesser potency may be, but are not limited to, somatic stem cells, tissue specific progenitor cells, primary or secondary cells. In some embodiments, the iPSCs are human iPSCs (hiPSCs).

In some embodiments, the cells comprise genetically modified cells. In practice genetically modified cells are engineered so as to synthesize the factors and the nucleic acids that promote cell differentiation into a given cell type, and/or promote tissue regeneration and/or tissue repairing and/or tissue healing.

Within the scope of the instant invention, the expression "genetically modified" is intended to refer to a cell that possesses one or more nucleotide substitution, addition or deletion in its genome and/or comprises one or more additional extra chromosomic nucleic acids encoding one or more factors interfering with the physiological outcome of the cell's fate. In certain embodiments, the genetically modified cells are of animal origin, preferably of mammal origin, more preferably of human origin.

In some embodiments, the genetically modified cells are engineered so as to allow the synthesis of one or more growth factor, transcription factor or RNAs involved cell differentiation into a given cell type, and/or in tissue regeneration and/or in tissue repairing and/or in tissue healing.

In certain embodiments, the cells' density is from about 10² to about 10¹⁶ cells per gram of the particulate material, preferably from about 10⁶ to about 10¹² cells per gram of the particulate material. Within the scope of the instant invention, the expression "from about 10² to about 10¹⁶ cells" encompasses 10², 5×10², 10³, 5×10³, 10⁴, 5×10⁴, 10⁵, 5×10⁵, 10⁶, 5×10⁶, 10⁷, 5×10⁷, 10⁸ 5×10⁸, 10⁹ 5×10⁹, 10¹⁰, 5×10¹⁰, 10¹¹, 5×10¹¹, 10¹², 5×10¹², 10¹³, 5×10¹³, 10¹⁴, 5×10¹⁴, 10¹⁵, 5×10¹⁵ and 10¹⁶ cells.

As used herein, a "culture medium" refers to the generally accepted definition in the field of cellular biology, *i.e.,* any medium suitable for promoting the growth of the cells of interest. Within the scope of the invention, the term "culture medium" includes a "proliferation culture medium" (referred to as MP) allows the cells to proliferate without promoting significant differentiation, and a "differentiation culture medium" (referred to as MD) promotes differentiation from one cell type to another cell type.

In some embodiments, a suitable culture medium may include a chemically defined medium, *i.e*., a nutritive medium only containing specified components, preferably components of known chemical structure.

In some embodiments, a chemically defined medium may be a serum-free and/or feeder-free medium. As used herein, a "serum-free" medium refers to a culture medium containing no added serum. As used herein, a "feeder-free" medium refers to a culture medium containing no added feeder cells.

A culture medium for use according to the invention may be an aqueous medium that may include a combination of substances such as one or more salts, carbon sources, amino acids, vitamins, minerals, reducing agents, buffering agents, lipids, nucleosides, antibiotics, cytokines, and growth factors.

Examples of suitable culture media include, without being limited to, RPMI medium, William's E medium, Basal Medium Eagle (BME), Eagle's Minimum Essential Medium (EMEM), Minimum Essential Medium (MEM), Dulbecco's Modified Eagles Medium (DMEM), Ham's F-10, Ham's F-12 medium, Kaighn's modified Ham's F-12 medium, DMEM/F-12 medium, and McCoy's 5A medium, which may be further supplemented with any one of the above mentioned substances.

In some embodiments, a culture medium according to the invention may be a synthetic culture medium such as the RPMI (Roswell Park Memorial Institute medium) or the CMRL-1066 (Connaught Medical Research Laboratory).

In one embodiment, proliferation medium may be any culture medium designed to support the growth of the cells known to one of ordinary skill in the art. As used herein, the proliferation medium is also called "growth medium". Examples of growth medium include, without limitation, RPMI, MEM, DMEM, IMDM, RPMI 1640, FGM or FGM-2, 199/109 medium, HamF10/HamF12 or McCoy's 5A. In a preferred embodiment, the proliferation medium is DMEM.

In practice, the culture parameters such as the temperature, the pH, the salinity, and the levels of O₂ and CO₂ may be adjusted accordingly to the standards established in the state of the art. Illustratively, the temperature for culturing the cells according to the invention may range from about 30°C to about 42°C, preferably from about 35°C to about 40°C, and more preferably from about 36°C to about 38°C. Within the scope of the invention, the expression "from about 30°C to about 42°C" encompasses 30°C, 31°C, 32°C, 33°C, 34°C, 35°C, 36°C, 37°C, 38°C, 39°C, 40°C, 41°C and 42°C.

In some embodiments, the level of CO₂ during the course of culture may be maintained constant and ranges from about 1% to about 10%, preferably from about 2.5% to about 7.5%. Within the scope of the invention, the expression "from about 1% to about 10%" encompasses 1, 2, 3, 4, 5, 6, 7, 8, 9 and 10%.

In some embodiments, the cells, in particular ASCs, are late passaged adipose-derived stem cells. As used herein, the term "late passages" means adipose-derived stem cells differentiated at least after passage 4. As used herein, the passage 4 refers to the fourth passage, *i.e.* the fourth act of splitting cells by detaching them from the surface of the culture vessel before they are resuspended in fresh medium. In one embodiment, late passaged adipose-derived stem cells are differentiated after passage 4, passage 5, passage 6 or more. In a preferred embodiment, cells, in particular ASCs, are differentiated after passage 4.

As used herein, the term "vessel" means any cell culture surface, such as for example a flask or a well-plate.

The initial passage of the primary cells was referred to as passage 0 (P0). According to the present invention, passage P0 refers to the seeding of cell suspension from the pelleted Stromal Vascular Fraction (SVF) on culture vessels. Therefore, passage P4 means that cells were detached 4 times (at P1, P2, P3 and P4) from the surface of the culture vessel (for example by digestion with trypsin) and resuspended in fresh medium.

In one embodiment, the cells of the invention, in particular ASCs, are cultured in proliferation medium up to the fourth passage. In one embodiment, the cells of the invention, in particular ASCs, are cultured in differentiation medium after the fourth passage. Accordingly, in one embodiment, at passages P1, P2 and P3, the cells of the invention, in particular ASCs are detached from the surface of the culture vessel and then diluted to the appropriate cell density in proliferation medium. Still according to this embodiment, at passage P4, cells, in particular ASCs, are detached from the surface of the culture vessel and then diluted to the appropriate cell density in differentiation medium. Therefore, according to this embodiment, at P4 the cells of the invention, in particular ASCs, are not resuspended and cultured in proliferation medium until they reach confluence before being differentiated (i.e. before being cultured in differentiation medium) but are directly resuspended and cultured in differentiation medium.

In certain embodiments, mature cells are obtained by differentiation of stem cells, genetically modified cells, or a mixture thereof, in an adapted differentiation medium. In some embodiments, the stem cells are mesenchymal stem cells, in particular adipose tissue-derived stem cells (ASCs).

In certain embodiments, the differentiation may result in the obtention of mature cells being selected in the group comprising, or consisting of, bone cells, brain cells, skin cells, breast cells, cells of the central nervous system, cervix cells, cells of the upper aero digestive tract, colorectal cells, endometrial cells, germ cells, bladder cells, kidney cells, laryngeal cells, liver cells, lung cells, esophageal cells, ovarian cells, pancreatic cells, pleural cells, prostate cells, ocular cells, small intestine cells, stomach cells, testicular cells, thyroid cells, and the like.

In practice, culture media, such as proliferation media may be supplemented with additional additives, commonly used in the field, so as to provide differentiating media. In some embodiments, the additional additives may be intended to promote differentiation, *i.e.,* but not limited to, osteogenesis, chondrogenesis, myogenesis, angiogenesis, epitheliogenesis, endotheliogenesis, adipogenesis. In some embodiments, the additional additives may be intended to promote osteogenesis and/or chondrogenesis. Non-limitative examples of suitable additional additives encompass growth factors, transcription factors, osteocytes activators, osteoblasts activators, osteoclasts inhibitors, chondrocytes activators, the likes and a mixture thereof.

In one embodiment, osteogenic differentiation of stem cells or genetically modified cells, in particular ASCs, is performed by culture of cells in osteogenic differentiation medium (MD). In one embodiment, the osteogenic differentiation medium comprises human serum. In a particular embodiment, the osteogenic differentiation medium comprises human platelet lysate (hPL). In one embodiment, the osteogenic differentiation medium does not comprise any other animal serum, preferably it comprises no other serum than human serum.

In one embodiment, the osteogenic differentiation medium comprises or consists of proliferation medium supplemented with dexamethasone, ascorbic acid and sodium phosphate. In one embodiment, the osteogenic differentiation medium further comprises antibiotics, such as penicillin, streptomycin, gentamycin and/or amphotericin B. In one embodiment, all media are free of animal proteins.

In one embodiment, the osteogenic differentiation medium comprises or consists of DMEM supplemented with L-alanyl-L-glutamine (Ala-Gin, also called 'Glutamax^{®}' or 'Ultraglutamine^{®}'), hPL, dexamethasone, ascorbic acid and sodium phosphate. In one embodiment, the osteogenic differentiation medium comprises or consists of DMEM supplemented with L-alanyl-L-glutamine, hPL, dexamethasone, ascorbic and sodium phosphate, and antibiotics, preferably penicillin, streptomycin, gentamycin and/or amphotericin B.

In one embodiment, the osteogenic differentiation medium comprises or consists of DMEM supplemented with L-alanyl-L-glutamine, hPL (about 5%, v/v), dexamethasone (about 1 µM), ascorbic acid (about 0.25 mM) and sodium phosphate (about 2.93 mM). In one embodiment, the osteogenic differentiation medium comprises or consists of DMEM supplemented with L-alanyl-L-glutamine, hPL (about 5%, v/v), dexamethasone (about 1 µM), ascorbic acid (about 0.25 mM) and sodium phosphate (about 2.93 mM), penicillin (about 100 U/mL) and streptomycin (about 100 µg/mL). In one embodiment, the osteogenic differentiation medium further comprises amphotericin B (about 0.1%).

In one embodiment, the osteogenic differentiation medium consists of DMEM supplemented with L-alanyl-L-glutamine, hPL (about 5%, v/v), dexamethasone (about 1 µM), ascorbic acid (about 0.25 mM) and sodium phosphate (about 2.93 mM). In one embodiment, the osteogenic differentiation medium comprises or consists of DMEM supplemented with L-alanyl-L-glutamine, hPL (about 5%, v/v), dexamethasone (about 1 µM), ascorbic acid (about 0.25 mM) and sodium phosphate (about 2.93 mM), penicillin (about 100 U/mL), streptomycin (about 100 µg/mL) and amphotericin B (about 0.1%).

Methods to control and assess the osteogenic differentiation are known in the art. For example, the osteo-differentiation of the cells or tissues may be assessed by staining of osteocalcin and/or phosphate *(e.g.,* with von Kossa); by staining calcium phosphate (*e.g.,* with Alizarin red); by magnetic resonance imaging (MRI); by measurement of mineralized matrix formation; or by measurement of alkaline phosphatase activity.

In another embodiment, the cells, in particular ASCs, are chondrogenic differentiated. In other words, in a preferred embodiment, the cells, in particular ASCs, are differentiated into chondrogenic cells. In still other words, in a preferred embodiment, the cells, in particular ASCs, are differentiated in chondrogenic medium. In a particular embodiment, the cells, in particular ASCs, are differentiated into chondrocytes.

In one embodiment, chondrogenic differentiation is performed by culture of the cells, in particular ASCs, in chondrogenic differentiation medium.

In one embodiment, the chondrogenic differentiation medium comprises or consists of DMEM, hPL, sodium pyruvate, ITS, proline, TGF-β1 and dexamethasone. In one embodiment, the chondrogenic differentiation medium further comprises antibiotics, such as penicillin, streptomycin, gentamycin and/or amphotericin B.

In one embodiment, the chondrogenic differentiation medium comprises or consists of proliferation medium supplemented with sodium pyruvate, ascorbic acid and dexamethasone. In one embodiment, the chondrogenic differentiation medium further comprises antibiotics, such as penicillin, streptomycin, gentamycin and/or amphotericin B. In one embodiment, the chondrogenic differentiation medium further comprises growth factors, such as IGF and TGF-β. In one embodiment, all media are free of animal proteins.

In one embodiment, the chondrogenic differentiation medium comprises or consists of DMEM supplemented with hPL, dexamethasone, ascorbic acid and sodium pyruvate. In one embodiment, the chondrogenic differentiation medium may further comprise proline and/or growth factors and/or antibiotics.

In one embodiment, the chondrogenic differentiation medium comprises or consists of DMEM, hPL (about 5%, v/v), dexamethasone (about 1 µM), sodium pyruvate (about 100 µg/mL), ITS (about IX), proline (about 40 µg/mL) and TGF-β1 (about 10 ng/mL).

Methods to control and assess the chondrogenic differentiation are known in the art. For example, the chondrogenic differentiation of the cells or tissues of the invention may be assessed by staining of Alcian Blue.

In another embodiment, the cells, in particular ASCs, are keratinogenic differentiated. In other words, in a preferred embodiment, the cells, in particular ASCs, are differentiated into keratinogenic cells. In still other words, in a preferred embodiment, the cells, in particular ASCs, are differentiated in keratinogenic medium. In a particular embodiment, the cells, in particular ASCs, are differentiated into keratinocytes.

In one embodiment, differentiation into keratinocytes are performed by culture of the cells, in particular ASCs, in keratinogenic differentiation medium.

In one embodiment, the keratinogenic differentiation medium comprises or consists of DMEM, hPL, insulin, KGF, hEGF, hydrocortisone and CaCl₂. In one embodiment, the keratinogenic differentiation medium further comprises antibiotics, such as penicillin, streptomycin, gentamycin and/or amphotericin B.

In one embodiment, the keratinogenic differentiation medium comprises or consists of DMEM, hPL (about 5%, v/v), insulin (about 5 µg/mL), KGF (about 10 ng/mL), hEGF (about 10 ng/mL), hydrocortisone (about 0.5 µg/mL) and CaCl₂ (about 1.5 mM).

Methods to control and assess the keratinogenic differentiation are known in the art. For example, the keratinogenic differentiation of the cells or tissues of the invention may be assessed by staining of Pankeratin or CD34.

In another embodiment, the cells, in particular ASCs, are endothelial differentiated. In still other words, in a preferred embodiment, the cells, in particular ASCs, are differentiated in endothelial medium. In a particular embodiment, the cells, in particular ASCs, are differentiated into endothelial cells.

In one embodiment, differentiation into endothelial cells are performed by culture of ASCs in endothelial differentiation medium.

In one embodiment, the endothelial differentiation medium comprises or consists of EBMTM-2 medium, hPL, hEGF, VEGF, R3-IGF-1, ascorbic acid, hydrocortisone and hFGFb. In one embodiment, the endothelial differentiation medium further comprises antibiotics, such as penicillin, streptomycin, gentamycin and/or amphotericin B.

In one embodiment, the endothelial differentiation medium comprises or consists of EBMTM-2 medium, hPL (about 5%, v/v), hEGF (about 0.5 mL), VEGF (about 0.5 mL), R3-IGF-1 (about 0.5 mL), ascorbic acid (about 0.5 mL), hydrocortisone (about 0.2 mL) and hFGFb (about 2 mL), reagents of the kit Clonetics^{™} EGM^{™}-2MV BulletKit^{™} CC-3202 (Lonza).

Methods to control and assess the endothelial differentiation are known in the art. For example, the endothelial differentiation of the cells or tissues of the invention may be assessed by staining of CD34.

In another embodiment, the cells, in particular ASCs, are myofibrogenic differentiated. In other words, in a preferred embodiment, the cells, in particular ASCs, are differentiated into myofibrogenic cells. In still other words, in a preferred embodiment, the cells, in particular ASCs, are differentiated in myofibrogenic medium. In a particular embodiment, the cells, in particular ASCs, are differentiated into myofibroblasts.

In one embodiment, differentiation into myofibrogenic cells are performed by culture of the cells, in particular ASCs, in myofibrogenic differentiation medium.

In one embodiment, the myofibrogenic differentiation medium comprises or consists of DMEM:F12, sodium pyruvate, ITS, RPMI 1640 vitamin, TGF-β1, Glutathione, MEM. In one embodiment, the myofibrogenic differentiation medium further comprises antibiotics, such as penicillin, streptomycin, gentamycin and/or amphotericin B.

In one embodiment, the myofibrogenic differentiation medium comprises or consists of DMEM:F12, sodium pyruvate (about 100 µg/mL), ITS (about IX), RPMI 1640 vitamin (about IX), TGF-β1 (about 1 ng/mL), Glutathione (about 1 µg/mL), MEM (about 0.1 mM).

Methods to control and assess the myofibrogenic differentiation are known in the art. For example, the myofibrogenic differentiation of the cells or tissues of the invention may be assessed by staining of α-SMA.

In another embodiment, the cells, in particular ASCs, are adipogenic differentiated. In other words, in a preferred embodiment, the cells, in particular ASCs, are differentiated into adipogenic cells. In still other words, in a preferred embodiment, the cells, in particular ASCs, are differentiated in adipogenic medium. In a particular embodiment, the cells, in particular ASCs, are differentiated into adipocytes.

In one embodiment, differentiation into adipocytes are performed by culture of the cells, in particular ASCs, in adipogenic differentiation medium.

In one embodiment, the adipogenic differentiation medium comprises or consists of DMEM, hPL, Dexamethasone, insulin, Indomethacin and IBMX. In one embodiment, the adipogenic differentiation medium further comprises antibiotics, such as penicillin, streptomycin, gentamycin and/or amphotericin B.

In one embodiment, the adipogenic differentiation medium comprises or consists of DMEM, hPL (about 5%), Dexamethasone (about 1 µM), insulin (about 5 µg/mL), Indomethacin (about 50 µM) and IBMX (about 0.5 mM).

Methods to control and assess the adipogenic differentiation are known in the art. For example, the adipogenic differentiation of the cells or tissues of the invention may be assessed by staining by Oil-Red.

In another embodiment, the cells, in particular ASCs, are neuronal differentiated. In other words, in a preferred embodiment, the cells, in particular ASCs, are differentiated into neural cells. In a particular embodiment, the cells, in particular ASCs, are differentiated into neural cells. In a specific embodiment, the cells, in particular ASCs, are differentiated into neurons. In another specific embodiment, the cells, in particular ASCs, are differentiated into glial cells.

In one embodiment, differentiation into neural cells are performed by culture of the cells, in particular ASCs, in neurons or glial cells differentiation medium.

Methods to control and assess the neural differentiation are known in the art. For example, the neural differentiation of the cells or tissues of the invention may be assessed according to the morphology, physiology, or global gene expression pattern. For instance, the neural differentiation of the cells or tissues of the invention may be assessed by the cell growth in length, by the development of a growth cone, and/or by staining of neuroectodermal stem cell markers including NESTIN, PAX6, and SOX2. Another method to control and assess the neural differentiation is to assess the electrophysiological profile of the differentiated cells.

In some embodiments, the cells, in particular ASCs, may be differentiated in any other suitable cell types, including hepatic cells, pancreatic cells, neural cells (or nervous cells), kidney cells, the likes, and any progenitor cells thereof. Differentiation protocols are readily available in the state of the art.

In some embodiments, cells are maintained in differentiation medium at least until they reach confluence, preferably between 70% and 100% confluence, more preferably between 80% and 95% confluence. In certain embodiments, cells are maintained in differentiation medium for at least about 5 days, preferably at least about 10 days, more preferably at least about 15 days. In one embodiment, cells are maintained in differentiation medium from about 5 days to about 30 days, preferably from about 10 days to about 25 days, more preferably from about 15 days to about 20 days. In one embodiment, differentiation medium is replaced every about 2 days. However, as it is known in the art, the cell growth rate from one donor to another could slightly differ. Thus, the duration of the differentiation and the number of medium changes may vary from one donor to another.

In one embodiment, cells are maintained in differentiation medium at least until formation of distinctive tissue depending on the differentiation medium used.

As used herein, the term "mature cells" refers to primary cells; differentiated cells, in particular differentiated stem cells or differentiated genetically modified cells. In practice, the mature cells have a phenotype that is identical or similar to naturally occurring cells originating from an individual's tissue or organ. In some embodiments, the mature cells are cells for which the differentiation potential been restricted to osteoblasts, osteocytes, chondroblasts, chondrocytes, keratinocytes, myofibroblasts, epithelial cells, endothelial cells, neural cells, adipocytes. In other words, the mature cells have a phenotype that is limited to a specific cell type.

In some embodiments, the mature cells are selected in the group comprising, or consisting of, bone cells, brain cells, skin cells, breast cells, neural cells, cervix cells, cells of the upper aero digestive tract, colorectal cells, endometrial cells, germ cells, bladder cells, kidney cells, laryngeal cells, liver cells, lung cells, esophageal cells, ovarian cells, pancreatic cells, pleural cells, prostate cells, ocular cells, small intestine cells, stomach cells, testicular cells, thyroid cells, and the likes.

As used herein, neural cells include, but are not limited to, cells of the central nervous system, such as neurons and glial cells.

In certain embodiments, the mature cells are selected from the group comprising or consisting of osteoblasts, osteocytes, chondroblasts, chondrocytes, keratinocytes, myofibroblasts, epithelial cells, endothelial cells, adipocytes, neural cells, and precursors thereof.

In practice, mature cells may be identified by their phenotype, in particular by expression of particular biomarkers; and/or by histological means, including cell staining; and/or by their specific physiological activity. Methods to identify mature cells according to the invention are well known from the state of the art. Illustratively, biomarkers' expression profile may be assessed at the nucleic acid level, *e.g.,* at the RNA level (*e.g.,* by RT-PCR (qPCR) analysis of RNA extracted from cultured cells with specific primers); and/or at the protein level *(e.g.,* by immunofluorescence analysis with markers-specific antibodies, such as Western blotting or ELISA; Fluorescent activated cell sorting (FACS); mass spectrometry; and enzymatic assays; and the like).

In some embodiments, the cells synthesized and/or secreted one or more factor(s) selected from the group comprising or consisting of BMPs, EGF, FGFs, HGF, IGF-1, OPG, SDF-1α, TGFB-1, TGFB-3, VEGF, including VEGFA and VEGFB, and a combination thereof.

In certain embodiments, the cells synthesized and/or secreted one or more factor(s) selected from the group comprising or consisting of OPG, SDF-1α, BMPR-1A, BMPR-2, FGFR-1, FGFR-2, TWIST-1, CSF-1, IGFR, RUNX2, TGFBR-1, and a combination thereof.

In some embodiments, the cells synthesized and/or secreted one or more factor(s) selected from the group comprising or consisting of SMAD-2, SMAD-3, SMAD-4, SMAD-5, AKT, ANG, ANGPT1, ANGPTL4, ANPEP, COL18A1, CTGF,CXCL1, EDN1, EFNA1, EFNB2, ENG, EPHB4, F3, FGF1, FGF2, FN1, HIF1A, ID1, IL6, ITGAV, JAG1, LEP, MMP14, MMP2, NRP1, PTGS1, SERPINE1, SERPINF1, TGFB1, TGFBR1, THBS1, THBS2, TIMP1, TIMP2, TIMP3, VEGFA, VEGFB, VEGFC, and a combination thereof.

In certain embodiments, the cells secreted IGF-1 and/or VEGF and/or SDF-1α and/or OPG.

In some embodiments, the cells secreted from about 0.1 ng to about 200 ng of IGF-1 per g (w/w) of the biomaterial, preferably from about 1 ng to about 150 ng per g of the biomaterial, more preferably from about 10 to about 80 ng per g of the biomaterial. As used herein, the term "biomaterial" is intended to refer to the 3-dimensional structure comprising the cells and the particulate material, embedded in an extracellular matrix. Within the scope of the invention, the expression "from about 0.1 ng to about 200 ng per g" includes about 0.1, 0.2, 0.3, 0.4, 0.5, 0.6, 0.7, 0.8, 0.9, 1, 2, 3, 4, 5, 6, 7, 8, 9, 10, 12, 14, 16, 18, 20, 22, 24, 26, 28, 30, 35, 40, 45, 50, 55, 60, 65, 70, 75, 80, 85, 90, 95, 100, 110, 120, 130, 140, 150, 160, 170, 180, 190 and 200 ng per g.

In certain embodiments, the cells secreted from about 0.1 ng to about 200 ng of VEGF per g (w/w) of the biomaterial, preferably from about 1 ng to about 150 ng per g of the biomaterial, more preferably from about 20 ng to about 100 ng per g of the biomaterial. Within the scope of the invention, the expression "from about 0.1 ng to about 200 ng per g" includes about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 160, 170, 180, 190 and 200 ng per g.

In some embodiments, the cells secreted from about 0.1 ng to about 400 ng of SDF-1α per g (w/w) of the biomaterial, preferably from about 1 ng to about 250 ng per g of the biomaterial, more preferably from about 10 ng to about 200 ng per g of the biomaterial. Within the scope of the invention, the expression "from about 0.1 ng to about 400 ng per g" includes about 0.1, 0.2, 0.3, 0.4, 0.5, 0.6, 0.7, 0.8, 0.9, 1, 2, 3, 4, 5, 6, 7, 8, 9, 10, 12, 14, 16, 18, 20, 22, 24, 26, 28, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390 and 400 ng per g.

In certain embodiments, the cells secreted from about 0.1 ng to about 100 ng of OPG per g (w/w) of the biomaterial, preferably from about 1 ng to about 50 ng per g of the biomaterial. Within the scope of the invention, the expression "from about 0.1 ng to about 100 ng per g" includes about 0.1, 0.2, 0.3, 0.4, 0.5, 0.6, 0.7, 0.8, 0.9, 1, 2, 3, 4, 5, 6, 7, 8, 9, 10, 12, 14, 16, 18, 20, 22, 24, 26, 28, 30, 35, 40, 45, 50, 55, 60, 65, 70, 75, 80, 85, 90, 95 and 100 ng per g.

In some embodiments, the cells secreted OPG.

In one embodiment, the particulate material of the invention is in form of particles. In one embodiment, particles may be beads, powder, spheres, microspheres, and the like.

In some embodiments, the particulate material of the invention is formed by a material that provides a structural support for the growth and propagation of cells. In one embodiment, particulate material is biocompatible, and comprises a natural or synthetic material, or a chemical-derivative thereof. Within the scope of the instant invention, "biocompatible" refers to the quality of not having toxic or injurious effects on the body.

In one embodiment, the particulate material of the invention is not structured to form a predefined 3D shape or scaffold, such as for example a cube. In one embodiment, the particulate material of the invention has not a predefined shape or scaffold. In one embodiment, the particulate material of the invention has not the form of a cube. In one embodiment, the particulate material is not a 3D scaffold. In certain embodiments, the particulate material is scaffold-free.

In some embodiments, the particulate material is selected from the group comprising or consisting of:
- an organic material, including demineralized bone matrix (DBM), gelatin, agar/agarose, alginates chitosan, chondroitin sulfate, collagen, elastin or elastin-like peptides (ELP), fibrinogen, fibrin, fibronectin, proteoglycans, heparan sulfate proteoglycans, hyaluronic acid, polysaccharides, laminins and cellulose derivatives;
- a ceramic material, including particles of calcium phosphate (CaP), calcium carbonate (CaCO₃), calcium sulfate (CaSO₄), or calcium hydroxide (Ca(OH)₂), or combinations thereof;
- a polymer, including polyanhydrides, polylactic acid (PLA), poly(lactic-co-glycolic acid) (PLGA), polyethylene oxide/ polyethylene glycol (PEO/PEG), poly(vinyl alcohol) (PVA), fumarate-based polymers such as, for example poly(propylene fumarate) (PPF) or poly(propylene fumarate-co-ethylene glycol) (P(PF-co-EG)), oligo(poly(ethylene glycol) fumarate) (OPF), poly (n-isopropylacrylamide) (PNIPPAAm), poly(aldehyde guluronate) (PAG), poly(n-vinyl pyrrolidone) (PNVP), or combinations thereof;
- a gel, including a self-assembling oligopeptide gel, a microgel, a nanogel, a particulate gel, a hydrogel, a thixotropic gel, a xerogel, a responsive gel, or combinations thereof;
- a creamer;
and any combination thereof.

In one embodiment, the particulate material according to the invention is added to the culture medium after differentiation of the cells, in particular when cells are sub-confluent or overconfluent, *i.e.,* when cells have reached confluence after differentiation. In one embodiment, the particulate material is added to the culture medium at least about 5 days after P4, preferably about 10 days after P4, more preferably about 15 days after P4. In one embodiment, the particulate material is added to the culture medium from about 5 days to about 30 days after P4, preferably from about 10 days to about 25 days after P4, more preferably from about 15 days to about 20 days after P4.

In some embodiment, the particulate material is gelatin, ceramic material, or a demineralized bone matrix (DBM).

In some embodiments, the particulate material of the invention is gelatin.

In one embodiment, the gelatin of the invention is animal gelatin, preferably mammal gelatin, more preferably porcine gelatin. As used herein, the term "porcine gelatin" may be replaced by "pork gelatin" or "pig gelatin". In one embodiment, the gelatin is porcine skin gelatin.

In one embodiment, the gelatin of the invention is in form of particles, beads, spheres, microspheres, and the like.

In one embodiment, the gelatin of the invention is not structured to form a predefined 3D shape or scaffold, such as for example a cube. In one embodiment, the gelatin of the invention has not a predefined shape or scaffold. In one embodiment, the gelatin of the invention has not the form of a cube. In one embodiment, the gelatin, preferably the porcine gelatin, is not a 3D scaffold. In one embodiment, the gelatin of the invention is a macroporous microcarrier.

In one embodiment, the gelatin of the invention is a macroporous microcarrier.

Examples of porcine gelatin particles include, but are not limited to, Cultispher^{®} G, Cultispher^{®} S, Spongostan and Cutanplast. In one embodiment, the gelatin of the invention is Cultispher^{®} G or Cultispher^{®} S.

In one embodiment, the gelatin, preferably the porcine gelatin, of the invention have a mean diameter of at least about 50 µm, preferably of at least about 75 µm, more preferably of at least about 100 µm, more preferably of at least about 130 µm. In one embodiment, the gelatin of the invention, preferably the porcine gelatin, have a mean diameter of at most about 1,000 µm, preferably of at most about 750 µm, more preferably of at most about 500 µm. In another embodiment, the gelatin of the invention, preferably the porcine gelatin, have a mean diameter of at most about 450 µm, preferably of at most about 400 µm, more preferably of at least most about 380 µm.

In one embodiment, the gelatin of the invention, preferably the porcine gelatin, has a mean diameter ranging from about 50 µm to about 1,000 µm, preferably from about 75 µm to about 750 µm, more preferably from about 100 µm to about 500 µm. In another embodiment, the gelatin of the invention, preferably the porcine gelatin, has a mean diameter ranging from about 50 µm to about 500 µm, preferably from about 75 µm to about 450 µm, more preferably from about 100 µm to about 400 µm. In another embodiment, the gelatin of the invention, preferably the porcine gelatin, have a mean diameter ranging from about 130 µm to about 380 µm.

Methods to assess the mean diameter of gelatin particles according to the invention are known in the art. Examples of such methods include, but are not limited to, granulometry, in particular using suitable sieves; sedimentometry; centrifugation techniques; laser diffraction; and images analysis, in particular by the means of a high-performance camera with telecentric lenses; and the like.

In one embodiment, gelatin is added to a mature cells' culture at a concentration ranging from about 0.1 cm³ to about 5 cm³ for a 150 cm² vessel, preferably from about 0.5 cm³ to about 4 cm³, more preferably from about 0.75 cm³ to about 3 cm³. In one embodiment, gelatin is added at a concentration ranging from about 1 cm³ to about 2 cm³ for a 150 cm² vessel. In one embodiment, gelatin is added at a concentration of about 1 cm³, 1.5 cm³ or 2 cm³ for a 150 cm² vessel. Within the scope of the invention, the expression "0.1 cm³ to about 5 cm³" encompasses 0.1 cm³, 0.2 cm³, 0.3 cm³, 0.4 cm³, 0.5 cm³, 0.6 cm³, 0.7 cm³, 0.8 cm³, 0.9 cm³, 1.0 cm³, 1.5 cm³, 2.0 cm³, 2.5 cm³, 3.0 cm³, 3.5 cm³, 4.0 cm³, 4.5 cm³ and 5.0 cm³.

In one embodiment, gelatin is added to a mature cells' culture at a concentration ranging from about 0.1 g to about 5 g for a 150 cm² vessel, preferably from about 0.5 g to about 4 g, more preferably from about 0.75 g to about 3 g. In one embodiment, gelatin is added at a concentration ranging from about 1 g to about 2 g for a 150 cm² vessel. In one embodiment, gelatin is added at a concentration of about 1 g, 1.5 g or 2 g for a 150 cm² vessel. Within the scope of the invention, the expression "0.1 g to about 5 g" encompasses 0.1 g, 0.2 g, 0.3 g, 0.4 g, 0.5 g, 0.6 g, 0.7 g, 0.8 g, 0.9 g, 1.0 g, 1.5 g, 2.0 g, 2.5 g, 3.0 g, 3.5 g, 4.0 g, 4.5 g and 5.0 g.

In another embodiment, the particulate material of the invention is a ceramic material.

In one embodiment, the ceramic material of the invention are particles of calcium phosphate (CaP), calcium carbonate (CaCO₃), calcium sulfate (CaSO₄), or calcium hydroxide (Ca(OH)₂), or combinations thereof.

Examples of calcium phosphate particles include, but are not limited to, hydroxyapatite (HA, Ca₁₀(PO₄)₆(OH)₂), tricalcium phosphate (TCP, Ca₃(PO₄)₂), α-tricalcium phosphate (α-TCP, (α-Ca₃(PO₄)₂), β-tricalcium phosphate (β-TCP, β-Ca₃(PO₄)₂), tetracalcium phosphate (TTCP, Ca₄(PO₄)₂O), octacalcium phosphate (Ca₈H₂(PO₄)₆.5H₂O), amorphous calcium phosphate (Ca₃(PO₄)₂), hydroxyapatite/β-tricalcium phosphate (HA/β-TCP), hydroxyapatite/tetracalcium phosphate (HA/TTCP), and the like.

In certain embodiments, the ceramic material of the invention comprises or consists of hydroxyapatite (HA), tricalcium phosphate (TCP), α-tricalcium phosphate (α-TCP), β-tricalcium phosphate (β-TCP), hydroxyapatite/β-tricalcium phosphate (HA/β-TCP), calcium sulfate (CaSO₄), or combinations thereof.

In certain embodiments, said ceramic material comprises calcium phosphate, preferably hydroxyapatite (HA) and/or β-tricalcium phosphate (β-TCP), more preferably particles of calcium phosphate.

In one embodiment, the ceramic particles of the invention are particles of hydroxyapatite (HA). In another embodiment, the ceramic particles of the invention are particles of β-tricalcium phosphate (β-TCP). In another embodiment, the ceramic particles of the invention are particles of hydroxyapatite/β-tricalcium phosphate (HA/β-TCP). In other words, in one embodiment, the ceramic particles of the invention are a mixture of hydroxyapatite and β-tricalcium phosphate particles (called HA/β-TCP particles). In one embodiment, the ceramic particles of the invention consist of hydroxyapatite particles and β-tricalcium phosphate particles (called HA/β-TCP particles).

In one embodiment, the particulate material, preferably the ceramic particles, more preferably HA, β-TCP and/or HA/β-TCP particles, are in form of granules, powder or beads. In one embodiment, the particulate material, preferably the ceramic particles, more preferably HA, β-TCP and/or HA/β-TCP particles, are in form of porous granules, powder or beads. In one embodiment, the particulate material, preferably the ceramic particles, more preferably HA, β-TCP and/or HA/β-TCP particles, are porous ceramic material. In one embodiment, the particulate material, preferably the ceramic particles, more preferably HA, β-TCP and/or HA/β-TCP particles, are powder particles. In a particular embodiment, the particulate material, preferably the ceramic particles, more preferably HA, β-TCP and/or HA/β-TCP particles, are in form of porous granules. In another particular embodiment, the particulate material, preferably the ceramic particles, more preferably HA, β-TCP and/or HA/β-TCP particles, are in form of powder. In one embodiment, the particulate material, preferably the ceramic particles, more preferably HA, β-TCP and/or HA/β-TCP particles, are not structured to form a predefined 3D shape or scaffold, such as for example a cube. In one embodiment, the particulate material, preferably the ceramic material of the invention is not a 3D scaffold. In one embodiment, the particulate material, preferably the ceramic material has not a predefined shape or scaffold. In one embodiment, the particulate material, preferably the ceramic material of the invention has not the form of a cube.

In one embodiment, the particulate material, preferably the ceramic particles of the invention, more preferably HA, β-TCP and/or HA/β-TCP particles, are larger than about 50 µm, preferably larger than about 100 µm. In one embodiment, the particulate material, preferably the ceramic particles of the invention, more preferably HA, β-TCP and/or HA/β-TCP particles, have a mean diameter larger than about 50 µm, preferably larger than about 100 µm.

In one embodiment, the particulate material, preferably the ceramic particles of the invention, more preferably HA, β-TCP and/or HA/β-TCP particles, have a mean diameter of at least about 50 µm, preferably of at least about 100 µm, more preferably of at least about 150 µm. In another embodiment, the particulate material, preferably the ceramic particles of the invention, more preferably HA, β-TCP and/or HA/β-TCP particles, have a mean diameter of at least about 200 µm, preferably of at least about 250 µm, more preferably of at least about 300 µm.

In another embodiment, the particulate material, preferably the ceramic particles of the invention, more preferably HA, β-TCP and/or HA/β-TCP particles, have a mean diameter of at most about 2,500 µm, preferably of at most about 2,000 µm, more preferably of at most about 1,500 µm. In one embodiment, the particulate material, preferably the ceramic particles of the invention, more preferably HA, β-TCP and/or HA/β-TCP particles, have a mean diameter of at most about 1,000 µm, 900 µm, 800 µm, 700 µm or 600 µm.

In one embodiment, the particulate material, preferably the ceramic particles of the invention, more preferably HA, β-TCP and/or HA/β-TCP particles, have a mean diameter ranging from about 50 µm to about 1,500 µm, preferably from about 50 µm to about 1,250 µm, more preferably from about 100 µm to about 1,000 µm. In one embodiment, the particulate material, preferably the ceramic particles of the invention, more preferably HA, β-TCP and/or HA/β-TCP particles, have a mean diameter ranging from about 100 µm to about 800 µm, preferably from about 150 µm to about 700 µm, more preferably from about 200 µm to about 600 µm.

In one embodiment, the HA/β-TCP particles have a mean diameter ranging from about 50 µm to about 1,500 µm, preferably from about 50 µm to about 1,250 µm, more preferably from about 100 µm to about 1,000 µm. In one embodiment, the HA and β-TCP particles have a mean diameter ranging from about 100 µm to about 800 µm, preferably from about 150 µm to about 700 µm, more preferably from about 200 µm to about 600 µm.

In practice, the measure of the mean sizes and diameters of particles may be performed by any suitable methods known in the state of the art, or a method adapted therefrom. Non-limiting examples of such methods include atomic force microscopy (AFM), transmission electron microscopy (TEM), scanning electron microscopy (SEM), dynamic light scattering (DLS).

In some embodiments, the ratio between HA and β-TCP (HA/β-TCP ratio) in the particles ranges from about 0/100 to about 100/0, including about 100/0, 90/10, 80/20, 70/30, 60/40, 50/50, 40/60, 30/70, 20/80, 10/90, or 0/100. In one embodiment, the ratio between HA and β-TCP (HA/β-TCP ratio) in the particles is about 60/40.

According to one embodiment, the quantity of particulate material, preferably ceramic particles, more preferably HA, β-TCP and/or HA/β-TCP particles, is optimal for providing a 3D structure to the biomaterial. In one embodiment, the particulate material, preferably the ceramic particles, more preferably HA, β-TCP and/or HA/β-TCP particles, are added at a concentration ranging from about 0.1 cm³ to about 5 cm³ for a 150 cm² vessel, preferably from about 0.5 cm³ to about 3 cm³, more preferably from about 1 cm³ to about 3 cm³. In a preferred embodiment, the particulate material, preferably the ceramic particles, more preferably HA, β-TCP and/or HA/β-TCP particles, are added at a concentration of about 1.5 cm³ to about 3 cm³ for a 150 cm² vessel.

In one embodiment, the particulate material, preferably the ceramic particles, more preferably HA, β-TCP and/or HA/β-TCP particles, are added at a concentration ranging from about 7×10⁻³ to 7×10⁻² cm³ per mL of medium. In one embodiment, the particulate material, preferably the ceramic particles, more preferably HA, β-TCP and/or HA/β-TCP particles, are added at a concentration ranging from about 3.3×10⁻³ to 3.3×10⁻² cm³ per cm² of vessel.

In another embodiment, the particulate material of the invention is demineralized bone matrix (DBM).

In one embodiment, DBM is of animal origin, preferably of mammal origin, more preferably of human origin. In a particular embodiment, human DBM is obtained by grinding cortical bones from human donors.

Methods to obtain DBM are well known in the art. For example, human bone tissue may firstly be defatted by acetone (*e.g.,* at 99%) bath during an overnight and then be washed in demineralized water during about 2 hours. Decalcification may be performed by immersion in HCL (*e.g.,* at about 0.6 N) during about 3 hours (*e.g.,* 20 mL solution per gram of bone) under agitation at room temperature. Then, demineralized bone powder may be rinsed with demineralized water during 2 hours and the pH is controlled. If the pH is too acid, DBM may be buffered with a phosphate solution (*e.g.,* at 0.1 M) under agitation. Finally, DBM may be dried and weighted. The DBM may be sterilized by Gamma irradiation following techniques known in the field, for example at about 25 kGray.

In one embodiment, the DBM is allogenic. In one embodiment, the DBM is homogenous. In another embodiment, the DBM is heterogeneous.

In one embodiment, DBM is in the form of particles, herein referred to as demineralized bone matrix particles or DBM particles. In one embodiment, the DBM particles have a mean diameter ranging from about 50 to about 2,500 µm, preferably from about 50 µm to about 1,500 µm, more preferably from about 50 µm to about 1,000 µm. In one embodiment, the DBM particles have a mean diameter ranging from about 100 µm to about 1,500 µm, more preferably from about 150 µm to about 1,000 µm. In one embodiment, the DBM particles have a mean diameter ranging from about 200 to about 1,000 µm, preferably from about 200 µm to about 800 µm, more preferably from about 300 µm to about 700 µm.

In some embodiments, the 3-dimensional structure obtained from the culture of mature cells and a particulate material comprises an extracellular matrix. In one embodiment, the extracellular matrix of the invention is produced and/or secreted by the mature cells, in particular the differentiated cells, preferably the differentiated ASCs.

As used herein, the term "extracellular matrix" (ECM) means a non-cellular three-dimensional macromolecular network. Matrix components of ECM bind to each other as well as cell adhesion receptors, thereby forming a complex network into which cells reside in tissues or in multidimensional structure as disclosed herein.

In one embodiment, the extracellular matrix of the invention comprises collagen, proteoglycans/glycosaminoglycans, elastin, fibronectin, laminin, and/or other glycoproteins. In a particular embodiment, the extracellular matrix of the invention comprises collagen. In another particular embodiment, the extracellular matrix of the invention comprises proteoglycans. In another particular embodiment, the extracellular matrix of the invention comprises collagen and proteoglycans. In one embodiment, the extracellular matrix of the invention comprises growth factors, proteoglycans, secreting factors, extracellular matrix regulators, and glycoproteins.

In one embodiment, the cells, preferably ASCs, and the particulate material, preferably the gelatin or the ceramic material, of the invention are embedded into the extracellular matrix.

In certain embodiments, the cellular extract is obtained from a culture of differentiated cells in the presence of a particulate material, wherein the cells and the particulate material were embedded in an extracellular matrix. In some embodiments, the cellular extract is obtained from a culture of osteo-differentiated cells in the presence of a particulate material, wherein the cells and the particulate material were embedded in an extracellular matrix. In certain embodiments, the cellular extract is obtained from a culture of osteo-differentiated MSCs, in particular osteo-differentiated ASCs, in the presence of a particulate material, wherein the cells and the particulate material were embedded in an extracellular matrix. In some embodiments, the cellular extract is obtained from a culture of osteo-differentiated ASCs, in the presence of a gelatin, wherein the cells and the gelatin were embedded in an extracellular matrix. In certain embodiments, the cellular extract is obtained from a culture of osteo-differentiated ASCs, in the presence of a ceramic material, wherein the cells and the ceramic material were embedded in an extracellular matrix.

In certain embodiments, the extracellular extract is obtained from a culture of differentiated cells in the presence of a particulate material, wherein the cells and the particulate material were embedded in an extracellular matrix. In some embodiments, the extracellular extract is obtained from a culture of osteo-differentiated cells in the presence of a particulate material, wherein the cells and the particulate material were embedded in an extracellular matrix. In certain embodiments, the extracellular extract is obtained from a culture of osteo-differentiated MSCs, in particular osteo-differentiated ASCs, in the presence of a particulate material, wherein the cells and the particulate material were embedded in an extracellular matrix. In some embodiments, the extracellular extract is obtained from a culture of osteo-differentiated ASCs, in the presence of a gelatin, wherein the cells and the gelatin were embedded in an extracellular matrix. In certain embodiments, the extracellular extract is obtained from a culture of osteo-differentiated ASCs, in the presence of a ceramic material, wherein the cells and the ceramic material were embedded in an extracellular matrix.

In certain embodiments, the cellular and/or extracellular extract is/are dehydrated and/or sterilized.

In certain embodiments, the composition is dehydrated. As used herein, the term "dehydrated" and the term "desiccated" are intended to be equivalent.

In some embodiments, dehydration is obtained by freeze-drying. Illustratively, freeze-drying, otherwise referred to as lyophilization, may be performed accordingly any one of the protocols disclosed in the state of the art, or a protocol adapted therefrom. In some embodiments, the freeze-drying of the composition is performed at a temperature of about -80°C, under vacuum.

In certain embodiments, sterilization is obtained by gamma-irradiation, preferably at a dose of about 7 kGy to about 45 kGy, more preferably at room temperature.

Within the scope of the invention, the expression "about 7 kGy to about 45 KGy" encompasses 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 and 45 kGy.

In some embodiments, the sterilization is obtained by gamma-irradiation at a dose of about 10 kGy to about 40 kGy.

Within the scope of the invention, the term "room temperature" is intended to refer to a temperature comprised from about 18°C to about 22°C, which encompasses 18°C, 19°C, 20°C, 21°C and 22°C. In some embodiments, room temperature is a temperature of about 20°C.

In some embodiments, the gamma-irradiation may be performed at a temperature below about 10°C, preferably on ice (about 0°C). Within the scope of the invention, a temperature below about 10°C encompasses 9.5°C, 8°C, 8.5°C, 8°C, 7.5°C, 7°C, 6.5°C, 6°C, 5°C, 4°C, 3°C, 2°C, 1°C, 0°C, -1°C, -2°C, -3°C, -4°C, -5°C, -10°C, -20°C, -30°C, - 40°C, -50°C, -60°C, -70°C and -80°C.

In practice, the gamma-irradiation may be performed for a duration that would depend from the amount (*e.g*., expressed in ng, µg, mg or g) of ingredients to be sterilized and/or the dose to be administered. In certain embodiments, the gamma-irradiation may be performed from about 10 sec to about 24 h, preferably from about 5 min (300 sec) to about 12h, more preferably, from about 10 min (600 sec) to about 3 h (10,800 sec). Within the scope of the invention, the expression "from about 10 sec to about 24 h" encompasses 10 sec, 15 sec, 20 sec, 25 sec, 30 sec, 35 sec, 40 sec, 45 sec, 50 sec, 55 sec, 1 min, 2 min, 3 min, 4 min, 5 min, 6 min, 7 min, 8 min, 9 min, 10 min, 15 min, 20 min, 25 min, 30 min, 35 min, 40 min, 45 min, 50 min, 55 min, 1 h, 1 h 30, 2 h, 2 h 30, 3 h, 3 h 30, 4 h, 4 h 30, 5 h, 5 h 30, 6 h, 7 h, 8 h, 9 h, 10 h, 11 h, 12 h, 13 h, 14 h, 15 h, 16 h, 17 h, 18 h, 19 h, 20 h, 21 h, 22 h, 23 hand 24 h.

As used herein, "pharmaceutically acceptable vehicle" refers to any solvent, dispersion medium, coating, antibacterial and/or antifungal agent, isotonic and absorption delaying agent and the like.

In one embodiment, the pharmaceutical composition comprises one or more pharmaceutically acceptable vehicle, such as emulsifiers, viscosity increasing agents, antimicrobial agents, antioxidants, preservatives, gelling agents, permeation enhancers, stabilizing agents, and the likes.

In practice, the pharmaceutically acceptable vehicle may comprise one or more ingredient(s) selected in a group of additives polypeptides; amino acids; lipids; and carbohydrates. Among carbohydrates, one may cite sugars, including monosaccharides, di-, tri-, tetra-, and oligosaccharides; derivatized sugars such as alditols, aldonic acids, esterified sugars and the like; and polysaccharides or sugar polymers.

Examples of suitable pharmaceutically acceptable vehicles may include polypeptides such as, *e.g.,* gelatin, casein, and the like.

In certain embodiments, the cancer is a solid cancer.

In some embodiments, the solid cancer is selected from the group comprising, or consisting of, a bone cancer, a brain cancer, a skin cancer, a breast cancer, a cancer of the central nervous system, a cancer of the cervix, a cancer of the upper aero digestive tract, a colorectal cancer, an endometrial cancer, a germ cell cancer, a bladder cancer, a kidney cancer, a laryngeal cancer, a liver cancer, a lung cancer, a neuroblastoma, an esophageal cancer, an ovarian cancer, a pancreatic cancer, a pleural cancer, a prostate cancer, a retinoblastoma, a small intestine cancer, a soft tissue sarcoma, a stomach cancer, a testicular cancer and a thyroid cancer.

In certain embodiments, the solid cancer is selected from the group comprising, or consisting of, bone cancer, including an osteosarcoma; a brain cancer, including a glioblastoma; and a skin cancer, including a melanoma.

In some embodiments, the inflammatory disease is selected from the group comprising, or consisting of, hepatitis, asthma, chronic peptic ulcer, Crohn's disease, dermatitis, periodontitis, arthritis, osteoarthritis, rheumatoid arthritis, sinusitis, tuberculosis, ulcerative colitis, and the likes.

In certain embodiments, the inflammation is associated with at least one symptom selected in the group consisting of pain, swelling, redness, edema, aching, tenderness, soreness, or any combination thereof; and/or wherein the inflammation is caused by, results in, or contributes to, injury, strain, infection, sprain, trauma, soreness, ache, fatigue, cancer, generalized joint pain, arthritis, osteoarthritis, rheumatoid arthritis, or any combination thereof.

In some embodiments, the cancer or the inflammation is associated with RANKL/RANK system deregulation, as disclosed, *e.g.,* by Ono et al. (Inflamm. Regener. 40, 2 (2020). https://doi.org/10.1186/s41232-019-0111-3).

In some embodiments, the cancer or the inflammation is associated with RANKL/RANK system activation.

In some embodiments, the cancer is associated with RANKL/RANK system deregulation.

In certain embodiments, cancers associated with RANKL/RANK system deregulation include, but are not limited to breast cancer, lung cancer, multiple myeloma, bone metastasis, and the likes.

In some embodiments, the inflammation is associated with RANKL/RANK system activation.

In certain embodiments, inflammatory diseases associated with RANKL/RANK system activation include, but are not limited to inflammatory bone loss, osteoporosis, postmenopausal osteoporosis, rheumatoid arthritis, periodontitis, skin inflammation, inflammation of the central nervous system, and the likes.

It is to be understood that the nature of the mature cells may be adapted to the tumor and/or the inflammation type to be prevented and/or treated. For example, skin cancer, such as melanoma, or skin inflammation, such as dermatitis, may benefit from cellular and/or extracellular extract(s) obtained from mature cells being selected from keratinocytes, epithelial cells, fibroblasts, and the like; whereas, bone cancer and osteoporosis may benefit from cellular and/or extracellular extract(s) obtained from mature cells being selected from osteoblasts, osteocytes, and the likes. On the other hand, joint inflammation, such as arthritis may benefit from cellular and/or extracellular extract(s) obtained from mature cells being selected from chondroblasts, chondrocytes, and the like.

In certain embodiments, the pharmaceutical composition is combined before use with any one of an isotonic aqueous solution; a scaffold material; another pharmaceutical composition; medical device; a material of biological origin; and any combination thereof.

In some embodiments, the pharmaceutical composition according to the invention may be formulated in any suitable form encompassed by the state in the art, *e.g.,* in the form of an injectable solution or suspension, a tablet, a coated tablet, a capsule, a syrup, a suppository, a cream, an ointment, a lotion, a gel and the like.

In some embodiments, the pharmaceutical composition of the instant invention may be rehydrated before administration. Illustratively, the pharmaceutical composition of the instant invention may be rehydrated with a sterile saline composition, in particular a sterile saline composition comprising from about 0.75% to about 1.25% NaCl, more preferably a sterile saline composition comprising from about 0.90% NaCl.

In some embodiments, the pharmaceutical composition according to the invention is to be formulated as a putty, an emollient, a cream, an ointment, a lotion, a gel, a salve, a controlled-release matrix, a liposomal or a lipid particle preparation, a microcapsule, or a nanocapsule, a suppository, a transdermal delivery system, or any combination thereof.

In some embodiments, the pharmaceutical composition is in the form of a semi solid. In some embodiments, the pharmaceutical composition is in the form of a paste, an ointment, a cream, a plaster or a gel. In some embodiments, the pharmaceutical composition may be in the form of a moldable paste or a film that can be manipulated and grafted.

In one embodiment, the pharmaceutical composition of the invention can be processed together with suitable excipients to the semi solid form, preferably the paste. Suitable excipients are, in particular, those excipients normally used to produce paste bases. Particularly suitable according to the invention are excipients normally used to produce gel-like paste bases, such as gel formers. Gel formers are substances which form gels with a dispersant such as water. Examples of gel formers of the invention are sheet silicates, carrageenan, xanthan, gum acacia, alginates, alginic acids, pectins, modified celluloses or poloxamers.

In one embodiment, the pharmaceutical composition in a semi solid form, preferably in the form of a paste, is ready for use. In another embodiment, the pharmaceutical composition in a semi solid form, preferably in the form of a paste, has to be extemporaneously produced.

In some embodiments, the miRNAs comprised in the cellular and/or extracellular extract(s) of the invention are encapsulated, *i.e.,* are immobilized in a vesicular system. In one embodiment, the encapsulation is a bilayer encapsulation. In another embodiment, the encapsulation is a single layer encapsulation. In still another embodiment, the encapsulation is a matrix encapsulation.

In one embodiment, the vesicles encapsulating the miRNAs are made of a biopolymer. In another embodiment, the vesicles encapsulating the miRNAs are extracellular vesicles. In a particular embodiment, the vesicles encapsulating the miRNAs are exosomes. Thus, in this embodiment, the cellular and/or extracellular extract(s) of the invention comprises miRNAs-encapsulating exosomes. In a specific embodiment, the exosomes are cells-derived exosomes, preferably exosomes from which the miRNAs are derived. In another specific embodiment, the exosomes are engineered exosomes.

Exosome engineering may be performed by any suitable methods known in the state of the art, or adapted therefrom. One may refer to, *e.g*., "Exosome engineering: Current progress in cargo loading and targeted delivery" (Fu et al., NanoImplant, 2020, Volume 20, 100261).

In certain embodiments, the pharmaceutical composition according to the invention may be combined with another cancer treatment, in particular, chemotherapy, radiotherapy and/or surgery, including tumor resection.

As used herein, the term "chemotherapy" refers to a drug treatment that uses chemicals to kill fast-growing cells, in particular cancer cells.

Non-limitative examples of chemotherapy agents include acalabrutinib, alectinib, alemtuzumab, anastrozole, avapritinib, avelumab, belinostat, bevacizumab, bleomycin, blinatumomab, bosutinib, brigatinib, carboplatin, carmustine, cetuximab, chlorambucil, cisplatin copanlisib, cytarabine, daunorubicin, decitabine, dexamethasone, docetaxel, doxorubicin, encorafenib, erdafitinib, etoposide, everolimus, exemestane, fludarabine, 5-fluorouracil, gemcitabine, ifosfamide, imatinib Mesylate, leuprolide, lomustine, mechlorethamine, melphalan, methotrexate, mitomycin, nelarabine, paclitaxel, pamidronate, panobinostat, pralatrexate, prednisolone, ofatumumab, rituximab, temozolomide, topotecan, tositumomab, trastuzumab, vandetanib, vinblastine, vincristine, vorinostat, zanubrutinib, and the likes.

In certain embodiments, the pharmaceutical composition according to the invention may be combined with another anti-inflammatory treatment.

Non-limitative examples of anti-inflammatory agents include aspirin, celecoxib, diclofenac, etoricoxib, ibuprofen, indomethacin, mefenamic acid, naproxen, oxaprozin, piroxicam, and the likes.

It is to be understood that the pharmaceutical composition according to the invention may be administered, before, concomitantly or after the cancer and/or anti-inflammatory treatment.

In some embodiments, an effective amount of said cellular and/or extracellular extract, as an active agent, is administered to said individual in need thereof. Within the scope of the instant invention, an "effective amount" refers to the amount of said cellular and/or extracellular extract(s), as an active agent, that alone stimulates the desired outcome, *i.e.* alleviates or eradicates the symptoms of cancer and/or inflammation.

Within the scope of the instant invention, the effective amount of the cellular and/or extracellular extract(s), as an active agent, to be administered may be determined by a physician or an authorized person skilled in the art and can be suitably adapted within the time course of the treatment.

In certain embodiments, the effective amount to be administered may depend upon a variety of parameters, including the material selected for administration, whether the administration is in single or multiple doses, and the individual's parameters including gender, age, physical condition, size, weight, and the severity of the disorder, *i.e.,* cancer and/or inflammation.

In certain embodiments, an effective amount of the cellular and/or extracellular extract(s), as an active agent, may comprise from about 0.001 mg to about 3,000 mg, per dosage unit, preferably from about 0.05 mg to about 100 mg, per dosage unit.

Within the scope of the instant invention, from about 0.001 mg to about 3,000 mg includes, from about 0.001 mg, 0.002 mg, 0.003 mg, 0.004 mg, 0.005 mg, 0.006 mg, 0.007 mg, 0.008 mg, 0.009 mg, 0.01 mg, 0.02 mg, 0.03 mg, 0.04 mg, 0.05 mg, 0.06 mg, 0.07 mg, 0.08 mg, 0.09 mg, 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850mg, 900mg, 950mg, 1,000 mg, 1,100 mg, 1,150 mg, 1,200 mg, 1,250 mg, 1,300 mg, 1,350 mg, 1,400 mg, 1,450 mg, 1,500 mg, 1,550 mg, 1,600 mg, 1,650 mg, 1,700 mg, 1,750 mg, 1,800 mg, 1,850 mg, 1,900 mg, 1,950 mg, 2,000 mg, 2,100 mg, 2,150 mg, 2,200 mg, 2,250 mg, 2,300 mg, 2,350 mg, 2,400 mg, 2,450 mg, 2,500 mg, 2,550 mg, 2,600 mg, 2,650 mg, 2,700 mg, 2,750 mg, 2,800 mg, 2,850 mg, 2,900 mg, 2,950 mg and 3,000 mg, per dosage unit.

In certain embodiments, the cellular and/or extracellular extract(s), as an active agent, may be at dosage levels sufficient to deliver from about 0.001 mg/kg to about 100 mg/kg, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day.

In certain embodiments, each dosage unit may be administered three times a day, two times a day, once a day, every other day, every three days, every week, every two weeks, every three weeks, or every four weeks.

In certain embodiments, the therapeutic treatment encompasses an administration of a plurality of dosage units, including two, three, four, five, six, seven, eight, nine, ten, eleven, twelve, thirteen, fourteen, or more administrations.

According to one embodiment, the pharmaceutical composition, medicament or medical device of the invention is to be administered by any suitable route, including enteral (*e.g.,* oral), parenteral, intravenous, intramuscular, intra-arterial, intramedullary, intrathecal, subcutaneous, intraventricular, transdermal, intradermal, rectal, intravaginal, intraperitoneal, topical, mucosal, nasal, buccal, sublingual; by intratracheal instillation, bronchial instillation, and/or inhalation; and/or as an oral spray, nasal spray, and/or aerosol.

In one embodiment, the pharmaceutical composition, medicament or medical device is to be administered at the site of the tissue disorder, in particular the tumor and/or the inflammation site. In certain embodiments, the pharmaceutical composition, medicament or medical device of the invention may be administered locally, *e.g.,* by injection, during surgery, in particular during invasive surgery.

In certain embodiments, the pharmaceutical composition is to be formulated as an injectable solution suitable for parenteral administration.

In some embodiments, the pharmaceutical composition according to the invention is to be refrigerated at a temperature of below about 0°C for storage. As used herein, the term "temperature of below about 0°C" includes, with being limited to 0°C, -1°C, -2°C, -3°C, -4°C, -5°C, -10°C, -20°C, -30°C, -40°C, -50°C, -60°C, -70°C and -80°C.

Another aspect of the invention relates to a medical device comprising a pharmaceutical composition according to the invention.

In some embodiments, the medical device is an implant. In some embodiments, the implant may be in the form of an organic or inorganic scaffold. In certain embodiments, the implant is resorbable.

The invention further pertains to an implant comprising a multi-dimensional biomaterial according to the instant disclosure. In some embodiments, the implant is allogeneic. In certain embodiments, the implant is autologous. In some embodiments, the implant is xenogeneic. In certain embodiments, the implant is lyophilized and sterilized, preferably sterilized by gamma-irradiation.

In certain embodiments, the medical device is a dressing for local application. In some embodiments, the dressing may comprise woven or non-woven fabrics. In some embodiments, the medical device is coated by or with the composition according to the present invention.

In certain embodiments, the medical device according to the invention is configured to allow the controlled release of the pharmaceutical composition. In some embodiments, the medical device is in the form of a patch.

Uses and methods may be performed *in vivo* or *ex vivo.*

The invention also pertains to the use of a pharmaceutical composition for the preparation and/or the manufacture of a medicament for the prevention and/or the treatment of cancer and/or inflammation, comprising:
- (i) a cellular and/or extracellular extract(s) obtained from a scaffold-free 3-dimensional culture of mature cells and a particulate material, wherein the mature cells secreted an extracellular matrix, and wherein the mature cells and the particulate material were embedded in the extracellular matrix; and
- (ii) a pharmaceutically acceptable excipient.

Another aspect of the invention relates to a method for the prevention and/or the treatment of cancer and/or inflammation, in an individual in need thereof, comprising administering a therapeutically effective amount of a pharmaceutical composition comprising:
- (i) a cellular and/or extracellular extract(s) obtained from a scaffold-free 3-dimensional culture of mature cells and a particulate material, wherein the mature cells secreted an extracellular matrix, and wherein the mature cells and the particulate material were embedded in the extracellular matrix; and
- (ii) a pharmaceutically acceptable excipient.

In certain embodiments, the pharmaceutical composition according to the invention is for use for modulating the biological activity of another pharmaceutical composition. In some embodiments, the said another pharmaceutical composition may comprise a chemotherapy agent and/or an anti-inflammatory agent.

In some embodiments, the pharmaceutical composition according to the invention is for modulating the expression of genes in mammal cells for cancer treatment.

In certain embodiments, the modulated gene(s) is/are selected from the group comprising, or consisting of, genes encoding AKT, BAX, FKHR/FOXO, ABL, CDK-2, CDK-4, CYCLIN D, CYCLIN E, HPV-E7, AURORA A, HPV-E6, MDM2, FAS, GPCR, GLI, HEDGEHOG, SMO, B-RAF, FOS/JUN, RAS, RTKS, MYC, B-CATENIN, RAR, SOX, WNT1, TALI, MLL, HOXS, MITF, EVI1, BCL6, and the likes, in human cells and their counterparts in mammals.

In certain embodiments, the modulated gene(s) is/are selected from the group comprising, or consisting of, genes encoding PI3K, BCL2, INPP4B, LKB1, PTEN, TSC1/TSC2, P15, P16, P57, RB, ARF, ATM/ATR, BRCA1, CHK1, CHK2, DNA-PK, FANCS, HIPK2, NBS1, P53, WT1, MUTYH, BLM, RECQL4, WRN, MMR, XPA, XPC, XPD, FBXW7, PTCH, SU(FU), EXT1, EXT2, INTEGRIN, NF1, NF2, VHL, FH, SDH, BMPR, SMAD2, SMAD3, TGFBR, MEN1, APC, AXIN, A-CATENIN, E-CADHERIN, WNT5A, GPC3, HRPT2, HPC1, and the likes, in human cells and their counterparts in mammals.

### BRIEF DESCRIPTION OF THE DRAWINGS

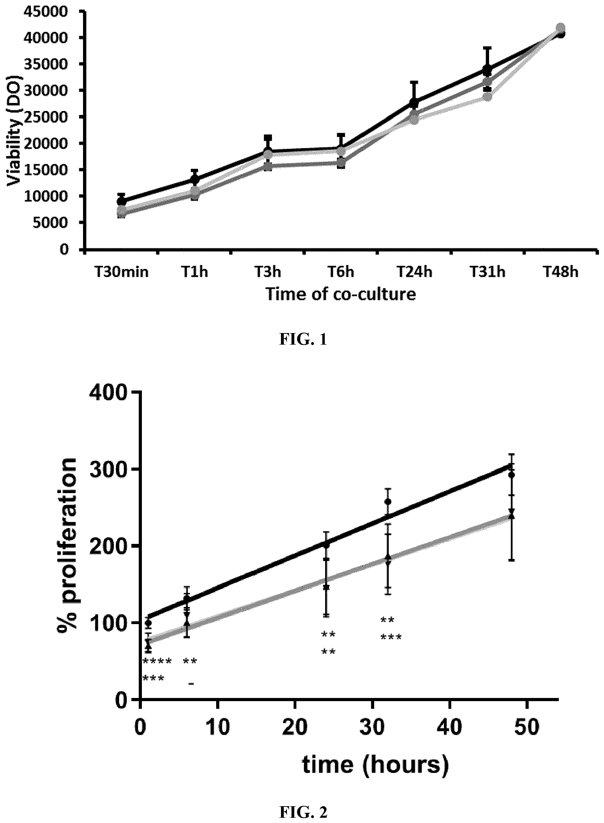

**Figure 1** is a plot showing the proliferation of human osteosarcoma cells H143B in the absence (black curve) or in the presence of 2.5 µg/ml (dark grey curve) and 25 µg/ml (light grey curve) of NVD002-Exosomes. The proliferation is expressed as viability (DO) with respect of the time of the co-culture of the cells and the exosomes.
**Figure 2** is a plot showing the linear regression of the loss of proliferation of human osteosarcoma cells H143B in the absence (black curve) or in the presence of 2.5 µg/ml (dark grey curve) and 25 µg/ml (light grey curve) of NVD002-Exosomes. Results are expressed as the % of viable cells vs negative control (without exosomes) at each time point. **: p<0.01; ***: p<0.005; ****: p<0.0001; -: no statistical difference.

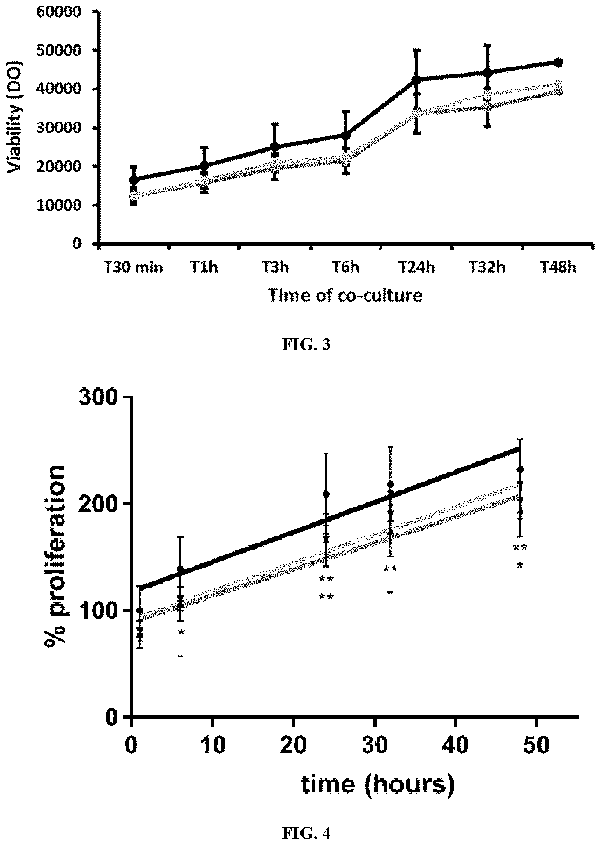

**Figure 3** is a plot showing the proliferation of human osteosarcoma cells H143B in the absence (black curve) or in the presence of 2.5 µg/ml (dark grey curve) and 25 µg/ml (light grey curve) of NVD003-Exosomes. The proliferation is expressed as viability (DO) with respect of the time of the co-culture of the cells and the exosomes.
**Figure 4** is a plot showing the linear regression of the loss of proliferation of human osteosarcoma cells H143B in the absence (black curve) or in the presence of 2.5 µg/ml (dark grey curve) and 25 µg/ml (light grey curve) of NVD003-Exosomes. Results are expressed as the % of viable cells vs negative control (without exosomes) at each time point. *: p<0.05; **: p<0.01; -: no statistical difference.

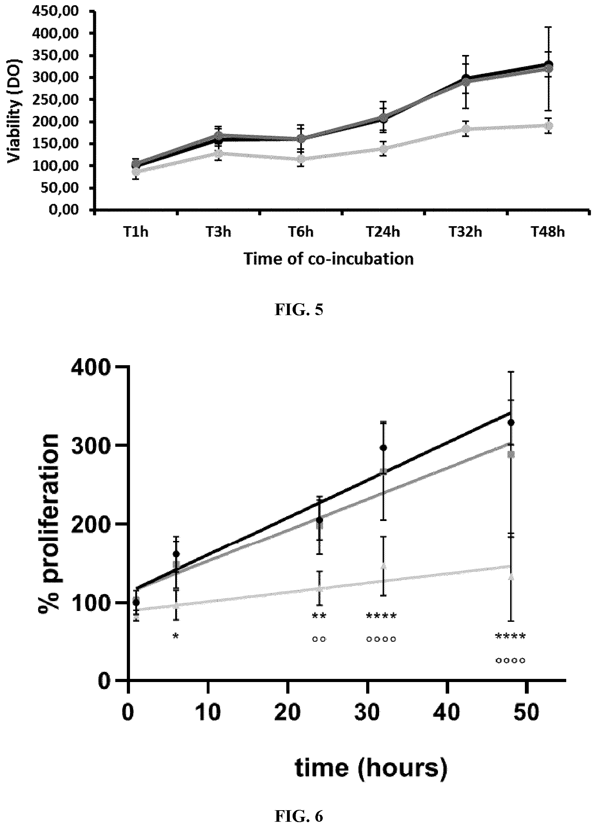

**Figure 5** is a plot showing the proliferation of human melanoma cells A375 in the absence (black curve) or in the presence of 2.5 µg/ml (dark grey curve) and 25 µg/ml (light grey curve) of NVD002-Exosomes. The proliferation is expressed as viability (DO) with respect of the time of the co-culture of the cells and the exosomes.
**Figure 6** is a plot showing the linear regression of the loss of proliferation of human melanoma cells A375 in the absence (black curve) or in the presence of 2.5 µg/ml (dark grey curve) and 25 µg/ml (light grey curve) of NVD002-Exosomes. Results are expressed as the % of viable cells vs negative control (without exosomes) at each time point. *: p<0.05; **: p<0.01; ****: p<0.0001; °°: p<0.01; °°°°: p<0.0001; -: no statistical difference.

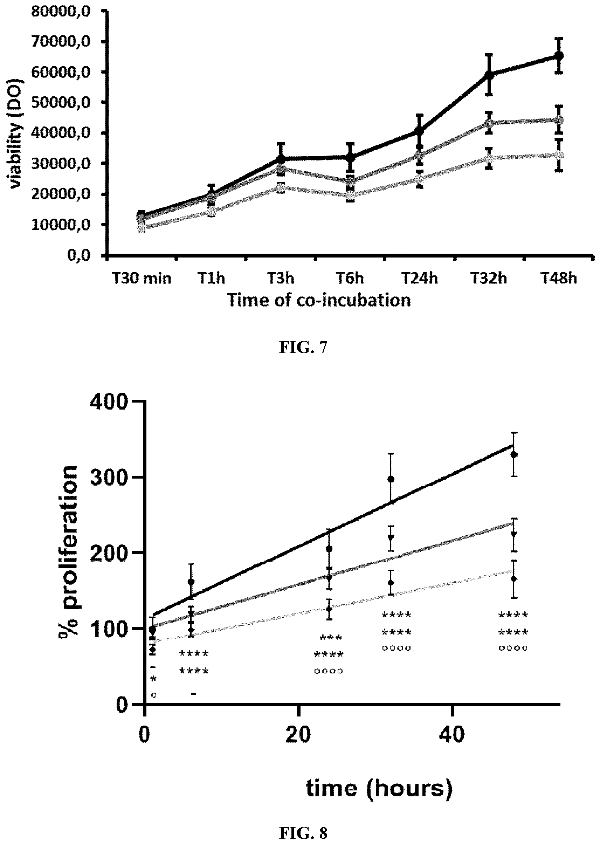

**Figure 7** is a plot showing the proliferation of human melanoma cells A375 in the absence (black curve) or in the presence of 2.5 µg/ml (dark grey curve) and 25 µg/ml (light grey curve) of NVD003-Exosomes. The proliferation is expressed as viability (DO) with respect of the time of the co-culture of the cells and the exosomes.
**Figure 8** is a plot showing the linear regression of the loss of proliferation of human melanoma cells A375 in the absence (black curve) or in the presence of 2.5 µg/ml (dark grey curve) and 25 µg/ml (light grey curve) of NVD003-Exosomes. Results are expressed as the % of viable cells vs negative control (without exosomes) at each time point. ***: p<0.005; ****: p<0.0001; °°°°: p<0.0001; -: no statistical difference.

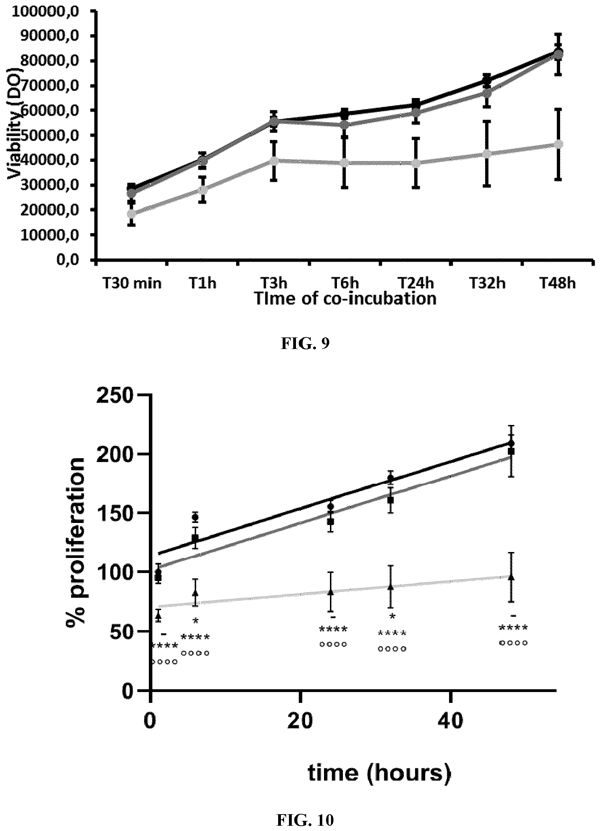

**Figure 9** is a plot showing the proliferation of human glioblastoma cells U87 in the absence (black curve) or in the presence of 2.5 µg/ml (dark grey curve) and 25 µg/ml (light grey curve) of NVD002-Exosomes. The proliferation is expressed as viability (DO) with respect of the time of the co-culture of the cells and the exosomes.
**Figure 10** is a plot showing the linear regression of the loss of proliferation of human glioblastoma cells U87 in the absence (black curve) or in the presence of 2.5 µg/ml (dark grey curve) and 25 µg/ml (light grey curve) of NVD002-Exosomes. Results are expressed as the % of viable cells vs negative control (without exosomes) at each time point. *: p<0.05; ****: p<0.0001; °°°°: p<0.0001; -: no statistical difference.

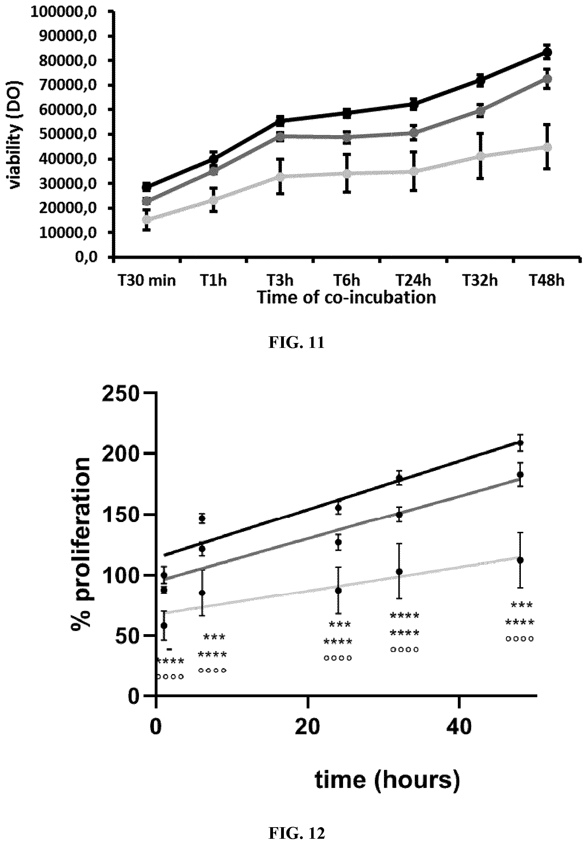

**Figure 11** is a plot showing the proliferation of human glioblastoma cells U87 in the absence (black curve) or in the presence of 2.5 µg/ml (dark grey curve) and 25 µg/ml (light grey curve) of NVD003-Exosomes. The proliferation is expressed as viability (DO) with respect of the time of the co-culture of the cells and the exosomes.
**Figure 12** is a plot showing the linear regression of the loss of proliferation of human glioblastoma cells U87 in the absence (black curve) or in the presence of 2.5 µg/ml (dark grey curve) and 25 µg/ml (light grey curve) of NVD003-Exosomes. Results are expressed as the % of viable cells vs negative control (without exosomes) at each time point. ***: p<0.005; ****: p<0.0001; °°°°: p<0.0001; -: no statistical difference.

### EXAMPLES

The present invention is further illustrated by the following examples.

### Example 1: Materials and Methods

### 1.1 Material

Three tumoral cell lines were used as target cells: H143B human osteosarcoma cells were obtained from ATCC^{®} (CRL-8303^{™}); A375 human melanoma cells were obtained from ATCC^{®} (CRL-1619^{™}); U87 human glioblastoma cells were obtained from ATCC^{®} (HTB-14^{™}).

### 1.2 Methods

### a) Preparation of NVD002 biomaterial

Human subcutaneous adipose tissues are harvested by lipo-aspiration (following the Coleman technique in the abdominal region) after informed consent and serologic screening.

Then, the lipoaspirate is digested by a collagenase solution (Serva Electrophoresis^{®} GmbH, Heidelberg, Germany) in Hanks' Balanced Salt Solution during 50-70 min at 37°C ± 1°C. The digestion is stopped by the addition of MP medium (proliferative medium consisting of Dulbecco's modified Eagle's medium, 4.5g/L Glucose/Ala-Gln (UltraGlutamine (Lonza^{®}) or Glutamax^{®} (Gibco^{®}), supplemented with 5% Human Platelet Lysate, 1% of penicillin/streptomycin. The digested adipose tissue is centrifuged (500×g, 10 min, at room temperature) and the supernatant is discarded. The pelleted Stromal Vascular Fraction (SVF) is re-suspended into MP medium and passed through a 200-500 µm mesh filter. The filtered cell suspension is centrifuged (500×g, 10 min, 20°C). The pellet containing the hASC is resuspended into MP medium. A sample of the cell suspension is used to seed one 75 cm² T-flask (Passage P0).

Between P0 and the fourth passage (P3/P4), cells are cultivated on T-flasks and fed with fresh proliferative medium (MP). This medium is composed of DMEM medium (4.5 g/L glucose and 4 mM Ala-Gln) supplemented with 5 % hPL (v/v), pH (7.2-7.4). Cells are passaged when reaching a confluence of about 80-90%. At each passage, cells are detached from their culture vessel with TrypLE^{®} (Select IX). TrypLE digestion is performed for 5-15 min at 37°C ± 2°C and stopped by the addition of MP medium (proliferative medium). Cells are then centrifuged (500×g, 5 min, room temperature), and re-suspended in MP medium (proliferative medium).

At the fourth passage (P3/P4), cells are seeded in re-closable cell culture flasks (150 cm²) and fed with osteogenic differentiation medium (MD). This medium is composed of proliferative medium (DMEM, Ala-Gln, hPL 5%) supplemented with dexamethasone (1µM), ascorbic acid (0.25 mM) and sodium phosphate (2.93mM). The volume of cell suspension is standardized to 70 ml per 150 cm² for the differentiation phase (MD medium).

When cells reach a confluence and if a morphologic change appears and if at least one osteoid nodule (un-mineralized, organic portion of the bone matrix that forms prior to the maturation of bone tissue) is observed in each flask, the 3-D induction can be started.

The culture vessels containing the confluent monolayer of adherent osteogenic cells are sprinkled with Cultispher particles (1.5 cc for a 150 cm² vessel). Few days after the addition of the Cultispher, the osteogenic cells and the particles dispersed become progressively entombed in mineralizing extracellular matrix.

Few days after, the osteogenic cells and the Cultispher particles start forming a large 3-dimensional patch (or few smaller patches) of partially mineralized translucid and malleable membrane detaching from each culture vessels. Regular medium exchanges are performed every 3 to 4 days during the 3D induction. Those medium exchanges are performed by carefully preventing removal of Cultispher particles and developing structure(s).

After about 15 days, the scaffold-free 3D culture (NVD-002) is developed and detached from the T-flasks. Cultures are maintained during 5 to 8 weeks after the addition of particles with medium change every 3-4 days.

### b) Preparation of NVD003 biomaterial

The protocol is identical to the preparation of NVD002 biomaterial (see section a) above), except for the 3-D induction of cells.

After being exposed to the osteogenic differentiation medium (MD), the culture vessels containing the confluent monolayer of adherent osteogenic cells are sprinkled with HA/β-TCP particles (3 cc for a 150 cm² vessel).

Few days after the addition of the HA/β-TCP, the osteogenic cells and the particles dispersed become progressively entombed in mineralizing extracellular matrix. Few days after, the osteogenic cells and HA/β-TCP particles start forming a large 3-dimensional patch (or few smaller patches) of partially mineralized brownish-yellow moldable putty detaching from each culture vessels. Regular medium exchanges are performed every 3 to 4 days during the 3D induction. Those medium exchanges are performed by carefully preventing removal of HA/β-TCP particles and developing structure(s).

After about 15 days, the scaffold-free 3D structure (NVD003 biomaterial) is developed and detached from the T-flasks. Cultures are maintained during 5 to 8 weeks after the addition of particles with medium change every 3-4 days.

### c) Isolation of exosomes

8 weeks after the addition of particles, NVD002 and NVD003 biomaterials were rinsed 3 times with PBS were placed in MD without hPL + 5% FBS depleted in exosomes for 72h. Supernatant was then harvested and centrifuged at 400×g for 5 minutes followed by 20 minutes at 2,000×g at 4°C. Supernatant was kept at 2/8°C for direct exosomes isolation. Isolated exosomes were then stored at -80C°.

Exosomes have been isolated by differential centrifugation from culture medium whereby larger "contaminants" are first excluded by pelleting out through increasing speeds of centrifugation before exosomes, small extracellular vesicles and even protein aggregates are pelleted at very high speeds (∼100,000×g).

### d) Proliferation assay

NVD003 and NVD002-derived exosomes from 3 donors were co-incubated in 96-wells plates with those three cell lines at 2.5 and 25 µg/ml for up to 72h at 37°C, 5% CO₂. A cell viability test (using the CellTiter-Glo^{®} Cell viability Assay from PROMEGA^{®}) was performed after 30 minutes to 48h of co-incubation, at minimum 5 different time points, to evaluate the proliferation of targeted cells. The CellTiter-Glo^{®} Luminescent Cell Viability Assay from PROMEGA^{®} is a homogeneous method to determine the number of viable cells in culture based on quantitation of the ATP present, which signals the presence of metabolically active cells). Experiments were performed in triplicate.

### e) Statistical analysis

Statistically significant differences between groups (with normal distribution) were tested by paired t-test and one-way analysis of variance with the Bonferroni post hoc test. Non-normal distributions of data were analyzed using the Kruskal-Wallis test. Statistical tests were performed with Prism GraphPad 2 (NIH). Statistical significance are as follows: *:p<0.05; **: p<0.01; ***: p<0.005; ****: p<0.0001.

### Example 2: In vitro effect of exosomes on human osteosarcoma cells (H143B)

### 2.1 Effect of NVD002-Exosomes

Proliferation curves of H143B cells cultured with exosomes showed a slightly lower level of viability than the control cells cultured without exosomes. In addition, a more marked effect was noted with the highest dose of exosomes (25 µg/ml vs 2.5 µg/ml) **(****Fig. 1****).**

Linear regression of the proliferation curves was calculated. Lower proliferation rates were found for cells cultured with exosomes, at both 2.5 and 25 µg/ml. A significant lower viability signal was found in cells co-cultured with exosomes at 2.5 and 25 µg/ml at 1, 24 and 32h of incubation and 2.5 µg/ml at 1, 6, 24 and 32h of incubation (p<0.01). **(****Fig. 2****).**

Although a higher slope was found for cells cultured without exosomes, it was associated with a higher viability level.

### 2.2 Effect of NVD003-Exosomes

Proliferation curves of H143B cells cultured with exosomes showed a slightly lower level of viability than the control cells cultured without exosomes **(****Fig. 3****).**

Linear regression of the proliferation curves was calculated. Lower proliferation rates were found for cells cultured with exosomes, at both 2.5 and 25 µg/ml. A significant lower viability signal was found in cells co-cultured with exosomes at 2.5 at 6, 24, 32 and 48h of incubation and 25 µg/ml only at 24h and 48h of incubation p<0.01). **(****Fig. 4****)**

Although a higher slope was found for cells cultured without exosomes, it was associated with a higher viability level.

### 2.3 Conclusion

In conclusion, NVD002- and NVD003-derived exosomes can reduce the proliferation of human osteosarcoma cell lines *in vitro.* A dose-response effect was observed.

### Example 3: In vitro effect of exosomes on human melanoma cells (A375)

### 3.1 Effect of NVD002-Exosomes

Although similar profile was found between cells cultured without exosomes and 2.5 µg/ml exosomes, proliferation curves of A375 cells cultured with 25 µg/ml exosomes showed a lower level of viability than the control cells cultured without exosomes **(****Fig. 5****).**

Linear regression of the proliferation curves was calculated. Lower proliferation rates were found for cells cultured with exosomes, at both 2.5 and 25 µg/ml. A significant lower viability signal was found in cells co-cultured with exosomes at 25 µg/ml at 1, 24, 32 and 48h of incubation (p<0.01). In addition, a significant lower viability signal was found at 24, 32 and 48h in cells treated with 25 µg/ml NVD002-Exo vs 2.5 µg/ml (p<0.01) **(****Fig. 6****).**

Although a higher slope was found for cells cultured without exosomes, it was associated with a higher viability level.

### 3.2 Effect of NVD003-Exosomes

Proliferation curves of A375 cells cultured with 2.5 and 25 µg/ml exosomes showed a lower level of viability than the control cells cultured without exosomes. This effect was more marked at 25 µg/ml exosomes than 2.5 µg/ml **(****Fig. 7****).**

Linear regression of the proliferation curves was calculated. Lower proliferation rates were found for cells cultured with exosomes, at both 2.5 and 25 µg/ml. A significant lower viability signal was found in cells co-cultured with exosomes at 25 µg/ml at each time point of incubation (p<0.01). A significant lower viability signal was found in cells co-cultured with exosomes at 2.5 µg/ml at 6, 24, 32 and 48h (p<0.05). A significant lower viability signal was found in cells co-cultured with exosomes at 25 µg/ml vs 2.5 µg/ml NVD003-Exo at 1, 24, 32, 48h (p<0.05) **(****Fig. 8****).**

Although a higher slope was found for cells cultured without exosomes, it was associated with a higher viability level.

### 3.3 Conclusion

In conclusion, NVD002- and NVD003-derived exosomes can reduce the proliferation of human melanoma cell lines *in vitro.* A dose-response effect was observed.

### Example 4: In vitro effect of exosomes on human glioblastoma cells (U87)

### 4.1 Effect of NVD002-Exosomes

Although similar profile was found between cells cultured without exosomes and 2.5 µg/ml exosomes, proliferation curves of U87 cells cultured with 25 µg/ml exosomes showed a lower level of viability than the control cells cultured without exosomes **(****Fig. 9****).**

Linear regression of the proliferation curves was calculated. Lower proliferation rates were found for cells cultured with exosomes, at both 2.5 and 25 µg/ml, with a more marked effect at 25 than 2.5 µg/ml. A significant lower viability signal was found in cells co-cultured with exosomes at 2.5 only at 6 and 32h (p<0.05) and 25 µg/ml at each time of incubation (p<0.0001). In addition, a significant lower viability signal was found for U87 cultured with 25 µg/ml NVD002-Exo vs 2.5 µg/ml at each tested timepoint (p<0.0001) **(****Fig. 10****).**

Although a higher slope was found for cells cultured without exosomes, it was associated with a higher viability level.

### 4.2 Effect of NVD003-Exosomes

Proliferation curves of U87 cells cultured with 2.5 and 25 µg/ml exosomes showed a lower level of viability than the control cells cultured without exosomes. This effect was more marked at 25 µg/ml exosomes than 2.5 µg/ml **(****Fig. 11****).**

Linear regression of the proliferation curves was calculated. Lower proliferation rates were found for cells cultured with exosomes, at both 2.5 and 25 µg/ml. A significant lower viability signal was found in cells co-cultured with exosomes at 2.5 µg/ml at 6, 24, 32 and 48h (p<0.0001). In addition, a significant lower viability signal was found for U87 cultured with 25 µg/ml NVD003-Exo vs 2.5 µg/ml at each tested timepoint (p<0.0001). **(****Fig. 12****).**

Although a higher slope was found for cells cultured without exosomes, it was associated with a higher viability level.

### 4.3 Conclusion

In conclusion, NVD002- and NVD003-derived exosomes can reduce the proliferation of human glioblastoma cell lines *in vitro.*

## Claims

1. A pharmaceutical composition for use in the prevention and/or the treatment of cancer and/or inflammation, comprising:
- (i) a cellular and/or extracellular extract(s) obtained from a scaffold-free 3-dimensional culture of mature cells and a particulate material, wherein the mature cells secreted an extracellular matrix, and wherein the mature cells and the particulate material were embedded in the extracellular matrix; and
- (ii) a pharmaceutically acceptable excipient.

2. The pharmaceutical composition for use according to claim **1,** wherein the extracellular extract comprises a matrisomal fraction and/or an exosomal fraction.

3. The pharmaceutical composition for use according to claim **1** or **2,** wherein the cellular and/or extracellular extract comprise(s) one or more miRNA(s).

4. The pharmaceutical composition for use according to any one of claims **1** to **3,** wherein the cells are selected from the group comprising or consisting of primary cells, stem cells, genetically modified cells, and a combination thereof.

5. The pharmaceutical composition for use according to any one of claims **1** to **4,** wherein the stem cells are mesenchymal stem cells, preferably adipose tissue-derived stem cells.

6. The pharmaceutical composition for use according to any one of claims **1** to **5,** wherein the mature cells are selected from the group comprising or consisting of osteoblasts, osteocytes, chondroblasts, chondrocytes, keratinocytes, myofibroblasts, epithelial cells, endothelial cells, adipocytes, neural cells, and precursors thereof.

7. The pharmaceutical composition for use according to any one of claims **1** to **6,** wherein the cells secreted OPG.

8. The pharmaceutical composition for use according to any one of claims **1** to **7,** wherein the particulate material is selected from the group comprising or consisting of:
- an organic material, including demineralized bone matrix (DBM), gelatin, agar/agarose, alginates chitosan, chondroitin sulfate, collagen, elastin or elastin-like peptides (ELP), fibrinogen, fibrin, fibronectin, proteoglycans, heparan sulfate proteoglycans, hyaluronic acid, polysaccharides, laminins and cellulose derivatives;
- a ceramic material, including particles of calcium phosphate (CaP), calcium carbonate (CaCO₃), calcium sulfate (CaSO₄), or calcium hydroxide (Ca(OH)₂), or combinations thereof;
- a polymer, including polyanhydrides, polylactic acid (PLA), poly(lactic-co-glycolic acid) (PLGA), polyethylene oxide/ polyethylene glycol (PEO/PEG), poly(vinyl alcohol) (PVA), fumarate-based polymers such as, for example poly(propylene fumarate) (PPF) or poly(propylene fumarate-co-ethylene glycol) (P(PF-co-EG)), oligo(poly(ethylene glycol) fumarate) (OPF), poly (n-isopropylacrylamide) (PNIPPAAm), poly(aldehyde guluronate) (PAG), poly(n-vinyl pyrrolidone) (PNVP), or combinations thereof;
- a gel, including a self-assembling oligopeptide gel, a microgel, a nanogel, a particulate gel, a hydrogel, a thixotropic gel, a xerogel, a responsive gel, or combinations thereof;
- a creamer;
and any combination thereof.

9. The pharmaceutical composition for use according to claim **8,** wherein said particulate material is gelatin, a ceramic material, or a demineralized bone matrix (DBM).

10. The pharmaceutical composition for use according to any one of claims **1** to **9,** wherein the cellular and/or extracellular extract is/are dehydrated and/or sterilized.

11. The pharmaceutical composition for use according to any one of claims **1** to **10,** wherein the cells are autologous, allogeneic, or xenogeneic.

12. The pharmaceutical composition for use according to any one of claims **1** to **11,** wherein the cancer is a solid cancer.

13. The pharmaceutical composition for use according to claim **12,** wherein the solid cancer is selected from the group comprising, or consisting of, a bone cancer, a brain cancer, a skin cancer, a breast cancer, a cancer of the central nervous system, a cancer of the cervix, a cancer of the upper aero digestive tract, a colorectal cancer, an endometrial cancer, a germ cell cancer, a bladder cancer, a kidney cancer, a laryngeal cancer, a liver cancer, a lung cancer, a neuroblastoma, an esophageal cancer, an ovarian cancer, a pancreatic cancer, a pleural cancer, a prostate cancer, a retinoblastoma, a small intestine cancer, a soft tissue sarcoma, a stomach cancer, a testicular cancer and a thyroid cancer.

14. The pharmaceutical composition for use according to claim **13,** wherein the solid cancer is selected from the group comprising, or consisting of, bone cancer, including an osteosarcoma; a brain cancer, including a glioblastoma; and a skin cancer, including a melanoma.

15. The pharmaceutical composition for use according to any one of claims **1** to **11,** wherein inflammation is associated with at least one symptom selected in the group consisting of pain, swelling, redness, edema, aching, tenderness, soreness, or any combination thereof; and/or wherein the inflammation is caused by, results in, or contributes to, injury, strain, infection, sprain, trauma, soreness, ache, fatigue, cancer, generalized joint pain, arthritis, osteoarthritis, rheumatoid arthritis, or any combination thereof.

16. The pharmaceutical composition for use according to any one of claims **1** to **15,** wherein the cancer or inflammation is associated with RANKL/RANK system activation.

17. The pharmaceutical composition for use according to any one of claims **1** to **16,** wherein it is combined before use with any one of an isotonic aqueous solution; a scaffold material; another pharmaceutical composition; medical device; a material of biological origin; and any combination thereof.

18. The pharmaceutical composition for use according to any one of claims **17,** wherein the said composition is to be formulated as a putty, an emollient, a cream, an ointment, a lotion, a gel, a salve, a controlled-release matrix, a liposomal or a lipid particle preparation, a microcapsule, or a nanocapsule, a suppository, a transdermal delivery system, or any combination thereof.
